# EUROPEAN PATENT SPECIFICATION

(11) **EP 2 582 433 B1**
(45) Date of publication and mention of the grant of the patent: **18.04.2018**
(21) Application number: 11729150.0
(22) Date of filing: 17.06.2011
(51) Int. Cl.: A61K 31/415, A61K 31/4155, A61P 11/00

(54) **UREIDO-PYRAZOLE DERIVATIVES FOR USE IN THE TREATMENT OF RHINOVIRUS INFECTIONS**
UREIDOPYRAZOLEINDERIVATE ZUR VERWENDUNG BEI DER BEHANDLUNG VON RHINOVIRUS-INFEKTIONEN
DÉRIVÉS D'URÉIDO-PYRAZOLE DESTINÉS À ÊTRE UTILISÉS DANS LE TRAITEMENT D'INFECTIONS PAR UN RHINOVIRUS

(30) Priority: 10.12.2010 WO PCT/GB2010/052066; 17.06.2010 GB 201010196
(43) Date of publication of application: 24.04.2013
(73) Proprietor: Respivert Limited, High Wycombe, Buckinghamshire HP12 4EG (GB)
(72) Inventor: CHARRON, Catherine Elisabeth, Imperial College London SW7 2AZ (GB); ITO, Kazuhiro, Imperial College London SW7 2AZ (GB); RAPEPORT, William Garth, Imperial College London SW7 2AZ (GB)
(74) Representative: Teuten, Andrew John
(86) International application number: PCT/GB2011/051139
(87) International publication number: WO 2011/158042

(56) References cited:
- EP-A1- 1 609 789
- WO-A1-2010/067130
- WO-A1-2010/067131
- WO-A1-2010/112936
- WO-A1-2011/070368
- WO-A2-01/19322
- WO-A2-2008/103692
- WO-A2-2010/015518
- WO-A2-2010/038085
- WO-A2-2010/038086
- US-A1- 2007 110 760
- SANDERSON MICHAEL P ET AL: "Syk: a novel target for treatment of inflammation in lung disease", INFLAMMATION & ALLERGY DRUG TARGETS, BENTHAM SCIENCE PUBLISHERS, vol. 8, no. 2, 1 June 2009 (2009-06-01), pages 87-95, XP009150784, ISSN: 1871-5281
- JOURNAL OF IMMUNOLOGY, vol. 177, no. 10, November 2006 (2006-11), pages 6859-6870, XP002653519, ISSN: 0022-1767
- INOUE D ET AL: "Mechanisms of mucin production by rhinovirus infection in cultured human airway epithelial cells", RESPIRATORY PHYSIOLOGY AND NEUROBIOLOGY, ELSEVIER, AMSTERDAM, NL, vol. 154, no. 3, 1 December 2006 (2006-12-01), pages 484-499, XP025120787, ISSN: 1569-9048, DOI: 10.1016/J.RESP.2005.11.006 [retrieved on 2006-12-01]
- BENTLEY J KELLEY ET AL: "Rhinovirus activates interleukin-8 expression via a Src/p110beta phosphatidylinositol 3-kinase/Akt pathway in human airway epithelial cells.", February 2007 (2007-02), JOURNAL OF VIROLOGY FEB 2007 LNKD- PUBMED:17121804, VOL. 81, NR. 3, PAGE(S) 1186 - 1194, XP002662158, ISSN: 0022-538X See abstract and page 1193: PP2 a c-SRC inhibitor inhibits IL-8 expression induced by rhinovirus. c-SRC is a potential target for teh treatment of rhinovirus associated diseases
- YAMAMOTO ET AL: "The orally available spleen tyrosine kinase inhibitor 2-[7-(3,4-dimethoxyphenyl)-imidazo[1,2-c]p yrimidin-5-ylamino]- nicotinamide dihydrochloride (BAY 61-3606) blocks antigen-induced airway inflammation in rodents", 20030101; 20030000, vol. 306, no. 3, 1 January 2003 (2003-01-01), pages 1174-1181, XP002333799,
- PATNAIK DEBASIS ET AL: "Identification of small molecule inhibitors of the mitotic kinase haspin by high-throughput screening using a homogeneous time-resolved fluorescence resonance energy transfer assay.", December 2008 (2008-12), JOURNAL OF BIOMOLECULAR SCREENING DEC 2008 LNKD- PUBMED:18978305, VOL. 13, NR. 10, PAGE(S) 1025 - 1034, XP002662169, ISSN: 1087-0571 See abstracts and results: methods for the screening of kinase inhibitors involving the use of FRET peptides and ATP

## Description

The present invention relates to compounds or compositions capable of inhibiting c-SRC and SYK activity for use in the treatment or prophylaxis of infection by human rhinovirus (HRV).

### Background

Such is the impact of the relentless onslaught of viruses on living organisms, that pathogenic viral infection has been one of the principle selection processes defining the course of human evolution. At one extreme, infections lead to long term damaging changes to the affected organ resulting, for example, in liver failure in the case of hepatitis B infection, or alternatively to the onset of malignant disease, such as uterine cancer, arising from papilloma virus infection. Alternatively viral infections may stimulate exacerbations of pre-existing, underlying chronic diseases; as manifested in asthma sufferers from rhinovirus infection or even precipitate acute, life-threatening disease, as occurs from multi-organ failure seen in high risk patients infected with influenza.

Many patients diagnosed with asthma or COPD (continue to suffer from uncontrolled symptoms and from exacerbations of their medical condition which can result in hospitalisation. This occurs despite the use of the most advanced, currently available treatment regimens, comprising of combination products of an inhaled corticosteroid and a long acting β-agonist. Data accumulated over the last decade indicates that a failure to effectively manage the underlying inflammatory component of the disease in the lung is the most likely reason that treatment is already poor in such cases or becomes increasingly ineffective. Given the established efficacy of corticosteroids as anti-inflammatory agents and, in particular, of inhaled corticosteroids in the treatment of asthma, these findings have provoked intense investigation. Resulting studies have identified that some environmental assaults invoke inflammatory changes in patients which prove to be *insensitive* to the actions of corticosteroids. An example of such a stimulated response arises from virally-mediated upper respiratory tract infections (URTI), which have particular significance in increasing morbidity associated with asthma and COPD.

Epidemiologic investigations have revealed a strong association between the presence of viral, upper respiratory tract infections and a substantial percentage of the exacerbations suffered by patients already diagnosed with chronic respiratory diseases. Some of the most compelling data in this regard derives from longitudinal studies of children suffering from asthma (Papadopoulos N.G., Papi A., Psarras S. and Johnston S.L., Paediatr. Respir. Rev., 2004, 5(3):255-260.). A variety of additional studies support the conclusion that a viral infection can precipitate exacerbations and increase disease severity. For example, experimental clinical infections with rhinovirus have been reported to cause bronchial hyper-responsiveness to histamine in asthmatics which is unresponsive to treatment with corticosteroids (Grunberg K., Sharon R.F., et al., Am. J. Respir. Crit. Care Med., 2001, 164(10):1816-1822.). Further evidence derives from the association observed between disease exacerbations in patients with cystic fibrosis and HRV infections (Wat D., Gelder C. et al., J. Cyst. Fibros., 2008, 7:320-328.). Also consistent with this body of data is the finding that respiratory viral infections, including rhinovirus, represent an independent risk factor that correlates negatively with the 12 month survival rate in paediatric, lung transplant recipients (Liu M., Worley S. et al., Transpl. Infect. Dis., 2009, 11(4):304-312.).

Clinical research indicates that the viral load is proportionate to the observed symptoms and complications and, by implication, to the severity of inflammation. For example, lower respiratory tract symptoms and bronchial hyper-responsiveness following experimental rhinovirus infection correlated significantly with virus load (Message S.D., Laza-Stanca V. et al., PNAS, 2008, 105(36):13562-13567.). Similarly, in the absence of other viral agents, rhinovirus infections were commonly associated with lower respiratory tract infections and wheezing, when the viral load was high in immunocompetent paediatric patients (Gerna G., Piralla A. et al., J. Med. Virol., 2009, 81(8):1498-1507.).

Significantly, it has been reported recently that prior exposure to rhinovirus reduced the cytokine responses evoked by bacterial products in human alveolar macrophages (Oliver B.G.G., Lim S. et al., Thorax, 2008, 63:519-525.). Additionally, infection of nasal epithelial cells with rhinovirus has been reported to promote adhesion of bacteria, including *S aureus* and *H influenzae* (Wang J.H., Kwon H.J. and Yong J.J., The Laryngoscope, 2009, 119(7):1406-1411.). Such cellular effects may contribute to the increased probability of patients suffering a lower respiratory tract infection following an infection in the upper respiratory tract. Accordingly, it is therapeutically relevant to focus on the ability of novel interventions to decrease viral load in a variety of *in vitro* systems, as a surrogate predictor of their benefit in a clinical setting.

High risk groups, for whom a rhinovirus infection in the upper respiratory tract can lead to severe secondary complications, are not limited to patients with chronic respiratory disease. They include, for example, the immunocompromised who are prone to lower respiratory tract infection, as well as patients undergoing chemotherapy, who face acute, life-threatening fever. It has also been suggested that other chronic diseases, such as diabetes, are associated with a compromised immunodefence response. This increases both the likelihood of acquiring a respiratory tract infection and of being hospitalised as a result. (Peleg A.Y., Weerarathna T. et al., Diabetes Metab. Res. Rev., 2007, 23(1):3-13; Kornum J.B., Reimar W. et al., Diabetes Care, 2008, 31(8):1541-1545.).

While viral, upper respiratory tract infections are a cause of considerable morbidity and mortality in those patients with underlying disease or other risk factors; rhinovirus infections also represent a significant healthcare burden in the general population and are a major cause of missed days at school and lost time in the workplace. (Rollinger J.M. and Schmidtke M., Med. Res. Rev., 2010, 1:42-92.). These considerations make it clear that novel medicines, possessing improved efficacy over current therapies, are urgently required to prevent and treat rhinovirus-mediated upper respiratory tract infections. In general the strategies adopted for the discovery of improved antiviral agents have targeted various proteins produced by the virus, as the point of therapeutic intervention. However, the wide range of rhinovirus serotypes makes this a particularly challenging approach to pursue and may explain why, at the present time, a medicine for the prophylaxis and treatment of rhinovirus infections has yet to be approved by any regulatory agency.

WO 2010/038086 A2 discloses compounds which are inhibitors of p38 mitogen-activated protein kinase (MAPK) enzymes, particularly alpha and gamma kinase sub-types, for use in the treatment of inflammatory diseases such as COPD.

WO 2010/067131 A1, WO 2010/067130 A1 and WO 2011/070368 A1 disclose p38 MAPK inhibitors for use as antiviral agents, for example in the treatment of conditions including influenza.

WO 2010/112936 A1 discloses a p38 MAPK inhibitor antiviral properties suitable for the prevention, treatment or amelioration of picornavirus infections, such as rhinovirus infection, influenza or respiratory syncitial virus.

EP 1,609,789 A1 discloses inhibitors of p38 kinase which are said to be useful for the treatment of conditions resulting from excessive cytokine production.

WO 01/19322 A2 discloses a CSBP/p38 inhibitor which is said to be useful for the treatment, including prophylaxis, of the common cold, or for example, respiratory viral infection caused by human rhinovirus infection (HRV).

Wang et al. (J. Immunol., 2006, 177(10):6859-6870) discloses that SYK is highly expressed by primary and cultured human airway epithelial cells and is said to be activated in response to infection with HRV16.

WO 2010/015518 A2 discloses substituted naphthyridines which are said to be SYK inhibitors for use in the treatment of various diseases including asthma, allergic rhinitis, rheumatoid arthritis, allergic dermatitis and COPD.

US 2007/110760 A1 discloses methods of treating, reducing the incidence of, and inhibiting metastasis formation of carcinomas, sarcomas, Epstein-Barr virus-induced malignancies, B cell proliferative disorders, and mast cell activation disorders, comprising administering to a subject a compound that is said to inhibit an interaction of a first protein and an immunoreceptor tyrosine-based activation motif (ITAM) of a second protein, and screening methods for identifying ITAM-inhibitory compounds and peptides.

WO 2008/103692 A2 discloses methods of identifying compounds which are said to be activators of lyn kinase.

Patnaik D. et al. (J. Biomolec. Screening, 2008, 13(10):1025-1034) discloses development of a high-throughput time-resolved fluorescence resonance energy transfer kinase assay for haspin which enables identification of inhibitors of haspin which are said to be useful as starting points for development of antimitotic anti-cancer therapeutics.

### Brief description of the Figures

The legends for the figures are as follows: **FP** = fluticasone propionate; **PI** = pleconaril; **BIRB** = **BIRB-796**; ** indicates p<0.01, * indicates p<0.05 vs. HRV control; **NT** = not treated.
**Figure 1****:** The effects of Reference Compounds 1 and 2, pleconaril and fluticasone propionate on extracellular HRV16 load.
**Figure 2****:** The effects of Reference Compounds 1 and 2, pleconaril and fluticasone propionate on HRV16 mRNA detected in cellular extract.
**Figure 3****:** The effects of Reference Compounds 1 and 2 and of pleconaril on HRV39 viral load in air-liquid interface cultured nasal epithelial cells.
**Figure 4****:** The effects of delaying treatment with Reference Compounds 1 and 2 on HRV extracellular load.
**Figure 5****:** The effects of Reference Compound 1, Reference Compound 2 and pleconaril on HRV39-induced IL-8 release in air-liquid interface culture nasal epithelial cells.
**Figure 6****:** Summary of the pathways and downstream consequences of RNA virus signalling.
**Figure 7****:** The effects of treatment with Reference Compound 2, pleconaril, **BIRB-796** and fluticasone propionate on interferon β induction (mRNA) by HRV16 in MRC-5 cells.
**Figure 8****:** The effects of Reference Compounds 1 and 2, **BIRB-796**, fluticasone propionate, BAY 61-036 and Dasatinib on HRV-16 induced activation of IRF-3.
**Figure 9****:** The effects of Reference Compound 1, Reference Compound 2, **BIRB-796**, fluticasone propionate, BAY 61-036 and Dasatinib on HRV-16 induced activation of NFκB.
**Figure 10****:** Plot of log [IC₅₀ value at c-SRC x IC₅₀ value at SYK] *versus* potency on HRV-16 virus load.

### Details of the invention

Virus entry to the host, the first step en route to virus propagation, is associated with the activation of a number of signalling pathways in the host cell which are believed to play a prominent role in the initiation of inflammatory processes (reviewed by Ludwig S., Signal Transduction, 2007, 7:81-88.) and those of viral propagation and subsequent release. One such mechanism, which has been determined to play a role in influenza virus propagation *in vitro,* is activation of the phosphoinositide 3-kinase / Akt pathway. The pathway has been described as being activated by the NS1 protein of the virus (Shin Y.K., Liu Q. et al., J. Gen. Virol., 2007, 88:13-18), and its inhibition is reported to reduce the titres of progeny virus (Ehrhardt C., Marjuki H. et al, Cell Microbiol., 2006, 8:1336-1348.).

Furthermore, the MEK inhibitor U0 126 has been reported to inhibit viral propagation without detection of resistant variants of the virus (Ludwig S., Wolff T. et al., FEBS Lett., 2004, 561:37-43.). More recently, studies targeting inhibition of SYK kinase have demonstrated that the enzyme plays an important role in mediating rhinovirus cell entry and virus-induced inflammatory responses, including ICAM-1 up-regulation (Sanderson M.P., Lau C.W. et al., Inflamm. Allergy Drug Targets, 2009, 8:87-95.). SYK activity is reported to be controlled by c-SRCas an upstream kinase in HRV infection (Lau C. et al., J. Immunol., 2008, 180:870-880.). A small number of studies have reported activation of cellular SRC (SRC1 or p60-SRC) or SRC family kinase in response to infection with viruses:
- Adenovirus has been reported to produce PI3 kinase mediated activation of Akt through a cSRC dependent mechanism,
- Rhinovirus-39 induced IL-8 production was suggested to depend upon SRC kinase activation in epithelial cells (Bentley J.K. and Newcomb D.C., J. Virol., 2007, 81:1186-1194.),
- Activation of SRC kinase was suggested to be involved in the induction of mucin production by rhinovirus-14 in epithelial cells and sub-mucosal glands (Inoue D. and Yamava M., Respir. Physiol. Neurobiol., 2006, 154:484-499.).

These induced responses in host cells which relate to virus propagation are offset by the initiation of processes which are intended to prevent or limit virus propagation and are focussed on interferons and their signalling pathways. Interferons are known to (i) promote the production of anti-viral proteins by infected cells, including enzymes which degrade viral nucleic acid; (ii) enhance the expression of antigens on the surface of infected cells promoting their recognition by cytotoxic T-cells; and (iii) activate cells involved in clearing viral infection, such as natural killer T cells and macrophages. Three different classes of interferons have been identified; type I (interferon α,β,ω), type II (interferon γ) and type III (interferon λ). Especially type I and III are known as anti-viral interferons.
Interestingly, viruses possess mechanisms to resist host immune responses. For example, human rhinovirus (HRV) inhibits interferon production by inhibition of IRF3 transcription factor (Peng T. et al., J. Virol., 2006, 80(10):5021-31; Ling Z. et al., J. Virol., 2009, 83(8):3734-42; Spann K.M., 2005, J. Virol., 79(9):5353-5362.).

Furthermore, research has suggested that epithelial cells from asthmatics have a compromised interferon response to rhinovirus infection that renders them less able to prevent infection (Wark P.A.B., Johnston S.L. et al., J.E.M., 2005, 201:937-947.). These results have been proposed as the basis for a novel therapeutic approach in which the host's compromised response is boosted by administration of exogenous interferon.

As well as direct anti-viral effects of type I and III interferons, type II interferons also interact with cells from the immune system to promote their accumulation and activation at specific sites. Interferon gamma has been reported to promote the chemotaxis of both neutrophils and macrophages following viral infection (Bonville C.A., Percopo C.M. et al., B. M. C. Immunol., 2009, 10:14; Crane M.J., Hokeness-Antonelli K.L. et al., J. Immunol., 2009, 183:2810-7.) and bacterial infection (Syn K., Salmon S.L. et al., Infect. Immun., 2007, 75:1196-1202; Ruan S., Young E. et al., Pulm. Pharmacol. Ther., 2006, 19:251-257.). The 10KDa-interferon-gamma-inducible protein, (IP)-10 (CXCL10) is known to play an important role in T-cell trafficking and homing, and recruitment of natural killer cells and macrophages (Nie C.Q., Bernard N.J., et al., Public Libraray of Science Pathog., 2009, 5, e1000369. doi:10.1371/Journal.ppat.1000369.). Intranasal administration of interferon α has been reported to protect ferrets and mice against infection with influenza virus (Kugel D., Kochs G. et al., J. Virol., 2009, 83:3843-3851; Van Hoeven N., Belser J.A. et al., J. Virol., 2009, 83:2851-2861.).

Thus clinical research suggests that there is a relationship between clinical viral load and symptoms and complications. For example, lower respiratory symptoms and bronchial hyper-responsiveness following experimental rhinovirus infection were significantly correlated with virus load (Message S.D., Laza-Stanca V. et al., PNAS, 2008, 105:13562-13567.). Similarly, rhinovirus infections were mostly associated with lower respiratory tract infections (in the absence of other viral agents) and wheezing, when viral load was high in immunocompetent pediatric patients (Gerna G., Piralla A. et al., J. Med. Virol., 2009, 81:1498-1507.). Interestingly, it has been reported recently that prior exposure to rhinovirus reduced the cytokine responses evoked in human alveolar macrophages to bacterial products (Oliver B.G.G., Lim S. et al., Thorax, 2008, 63:519-525). Additionally, infection of nasal epithelial cells with rhinovirus has been reported to promote adhesion of bacteria, including *S Aureus* and *H influenzae* (Wang, J.H., Kwon, H.J. et al., Laryngoscope, 2009, 119:1406-1411.). Such effects may contribute to the increased probability of lower respiratory tract infections in patients following an upper respiratory tract infection. Accordingly, it is appropriate and therapeutically relevant to focus on the ability of novel interventions to decrease viral load in a variety of systems *in vitro* as a surrogate predictor of clinical benefit in therapeutics.

The Inventors have discovered a correlation between inhibition of c-SRC and SYK activity against HRV, for example as measured by effect on viral load. SYK acts upstream of NFKB meaning that inhibition of c-SRC prevents HRV from exerting its deleterious activating effect on NFKB. c-SRC is a negative regulator of IRF3/7 meaning that inhibition of c-SRC prevents the virus from causing downregulation of the beneficial IRF3/7 activity. Thus we provide the following aspects of the invention:
- A compound or composition capable of inhibiting c-SRC and SYK activity for use in the treatment or prophylaxis of infection by human rhinovirus (HRV), wherein the compound or composition does not comprise *N*-(4-(4-(3-(3-*tert*-Butyl-1-*p*-tolyl-1H-pyrazol-5-yl) ureido)naphthalen-1-yloxy)pyridin-2-yl)-2-methoxyacetamide;
- Use of a compound or composition capable of inhibiting c-SRC and SYK activity for the manufacture of a medicament for the treatment or prophylaxis of infection by human rhinovirus (HRV), wherein the compound or composition does not comprise *N*-(4-(4-(3-(3-*tert*-Butyl-1-*p*-tolyl-1H-pyrazol-5-yl)ureido)naphthalen-1-yloxy)pyridin-2-yl)-2-methoxyacetamide;
- A method of screening for a candidate drug substance or substances intended to prevent or treat human rhinovirus (HRV) infection in a subject which comprises identifying one or more test substances which together or separately are capable of inhibiting c-SRC and SYK activity.

In addition, the present disclosure provides a compound of formula (I): 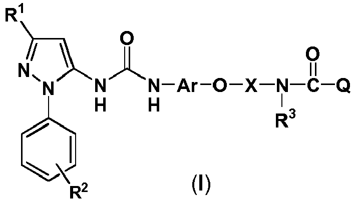
wherein R¹ is C₁₋₆ alkyl optionally substituted by a hydroxyl group;
R² is H or C₁₋₆ alkyl optionally substituted by a hydroxyl group;
R³ is H, C₁₋₆ alkyl or C₀₋₃ alkylC₃₋₆ cycloalkyl;
Ar is a naphthyl or a phenyl ring either of which may be optionally substituted by one or more (for example 1 or 2) groups independently selected from C₁₋₆ alkyl, C₁₋₆ alkoxy, amino, C₁₋₄ mono or di-alkyl amino;
X is 5 or 6 membered heteroaryl group containing at least one nitrogen atom and optionally including 1 or 2 further heteroatoms selected from O, S and N;
Q is selected from:
   a) a saturated or unsaturated, branched or unbranched C₁₋₁₀ alkyl chain, wherein at least one carbon (for example 1, 2 or 3 carbons, suitably 1 or 2, in particular 1 carbon) is replaced by a heteroatom selected from O, N, S(O)ₚ, wherein said chain is optionally, substituted by one or more groups (for example 1, 2 or 3 groups) independently selected from oxo, halogen, an aryl group, a heteroaryl group, a heterocyclyl group or a C₃₋₈ cycloalkyl group,
      each aryl, heteroaryl, heterocyclyl or C₃₋₈ cycloalkyl group bearing 0 to 3 substituents selected from halogen, hydroxyl, C₁₋₆ alkyl, C₁₋₆ alkoxy, C₁₋₆ haloalkyl, amino, C₁₋₄ mono or di-alkyl amino, C₁₋₄ mono or di-acyl amino, S(O)_{q}C₁₋₆ alkyl, C₀₋₆ alkylC(O)C₁₋₆ alkyl or C₀₋₆ alkylC(O)C₁₋₆ heteroalkyl,
      with the proviso that the atom linked directly to the carbonyl in -NR³C(O)- is not an oxygen or a sulfur atom; and
   b) a C₀₋₈ alkyl-heterocycle said heterocyclyl group comprising at least one heteroatom (for example 1, 2 or 3, suitably 1 or 2, in particular 1 heteroatom) selected from O, N, and S, and is optionally substituted by one, two or three groups independently selected from halogen, hydroxyl, C₁₋₆ alkyl, C₁₋₆ alkoxy, C₁₋₆ haloalkyl, amino, C₁₋₄ mono and di-alkyl amino, C₁₋₄ mono or di-acyl amino, S(O)_{q}C₁₋₆ alkyl, C₀₋₆ alkylC(O)C₁₋₆ alkyl or C₀₋₆ alkylC(O)C₁₋₆ heteroalkyl; and
p is 0, 1 or 2;
q is 0, 1 or 2
a pharmaceutically acceptable salt thereof, including all stereoisomers, tautomers and isotopic derivatives thereof for use in the treatment or prophylaxis of HRV infection and/or the exacerbation of respiratory disorders (such as asthma, COPD, bronchitis and/or cystic fibrosis) by rhinovirus infection.

Alkyl as used herein refers to straight chain or branched chain alkyl, such as, without limitation, methyl, ethyl, *n*-propyl, *iso*-propyl, butyl, *n*-butyl and *tert*-butyl. In one embodiment alkyl refers to straight chain alkyl.

Alkoxy as used herein refers to straight or branched chain alkoxy, for example methoxy, ethoxy, propoxy, butoxy. Alkoxy as employed herein also extends to embodiments in which the oxygen atom is located within the alkyl chain, for example -C₁₋₃ alkylOC₁₋₃ alkyl, such as -CH₂CH₂OCH₃ or -CH₂OCH₃. Thus in one embodiment the alkoxy is linked through carbon to the remainder of the molecule. In one embodiment the alkoxy is linked through oxygen to the remainder of the molecule, for example -C₀ alkylOC₁₋₆ alkyl. In one embodiment the disclosure relates to straight chain alkoxy.

Heteroalkyl as employed herein is intended to refer to a branched or straight chain alkyl wherein one or more, such as 1, 2 or 3 carbons are replaced by a heteroatom, selected from N, O or S(O)_{q}, wherein q represents 0, 1 or 2. The heteroatom may replace a primary, secondary or tertiary carbon, that is, for example, OH or NH₂ for CH₃, or NH or O or SO₂ for - CH₂- or N for a -CH- or a branched carbon group, as technically appropriate.

Haloalkyl as employed herein refers to alkyl groups having 1 to 6 halogen atoms, for example 1 to 5 halogens, such as *per* haloalkyl, in particular perfluoroalkyl, more specifically -CF₂CF₃ or CF₃.

C₁₋₄ mono or di-acyl amino is intended to refer to -NHC(O)C₁₋₃ alkyl and to (-NC(O)C₁₋₃ alkyl) C(O)C₁₋₃ alkyl) respectively.

C₁₋₄ mono or di-alkyl amino is intended to refer to -NHC₁₋₄ alkyl and -N(C₁₋₄ alkyl) (C₁₋₄ alkyl) respectively.

Aryl as used herein refers to, for example C₆₋₁₄ mono or polycyclic groups having from 1 to 3 rings wherein at least one ring is aromatic including phenyl, naphthyl, anthracenyl, 1,2,3,4-tetrahydronaphthyl and the like, such as phenyl and naphthyl.

Heteroaryl is a 6 to 10 membered aromatic monocylic ring or bicyclic ring system wherein at least one ring is an aromatic nucleus comprising one or more, for example 1, 2, 3 or 4 heteroatoms independently selected from O, N and S. Examples of heteroaryls include: pyrrole, oxazole, thiazole, isothiazole, imidazole, pyrazole, isoxazole, pyridine, pyridazine, pyrimidine, pyrazine, benzothiophene, benzofuran, or 1, 2, 3 and 1, 2, 4 triazole.

Heterocyclyl as employed herein refers to a 5 to 6 membered saturated or partially unsaturated non-aromatic ring comprising one or more, for example 1, 2, 3 or 4 heteroatoms independently selected from O, N and S optionally one or two carbons in the ring may bear an oxo substituent. The definition of C₅₋₆ heterocycle as employed herein refers to a is a 5 to 6 membered saturated or partially unsaturated non-aromatic carbocyclic ring comprising one or more, for example 1, 2, 3 or 4 heteroatoms independently selected from O, N and S, wherein each heteroatom replaces a carbon atom and optionally one or two carbons may bear an oxo substituent. Clearly any valancies of a heteroatom not employed in forming or retaining the ring structure may be filled by hydrogen or a substituent, as appropriate. Thus substituents on heterocycles may be on carbon or on a heteroatom, such as N as appropriate. Examples of heterocycles and C₅₋₆ heterocycles include pyrroline, pyrrolidine, tetrahydrofuran, tetrahydrothiophene, pyrazoline, imidazoline, pyrazolidine, imidazolidine, oxoimidazolidine, dioxolane, thiazolidine, isoxazolidine, pyran, dihydropyran, piperidine, piperazine, morpholine, dioxane, thiomorpholine and oxathiane.

Halogen includes fluoro, chloro, bromo or iodo, in particular fluoro, chloro or bromo, especially fluoro or chloro.

Oxo as used herein refers to C=O and will usually be represented as C(O).

C₃₋₈ cycloalkyl as employed herein is intended to refer to a saturated or partially unsaturated non-aromatic ring containing 3 to 8 carbon atoms.

C₁₋₁₀ alkyl includes C₂, C₃, C₄, C₅, C₆, C₇, C₈ or C₉ as well as C₁ and C₁₀

C₀₋₈ alkyl includes C₁, C₂, C₃, C₄, C₅, C₆, or C₇ as well as C₀ and C₈.

In relation to a saturated or unsaturated, branched or unbranched C₁₋₁₀ alkyl chain, wherein at least one carbon (for example 1, 2 or 3 carbons, suitably 1 or 2, in particular 1) is replaced by a heteroatom selected from O, N, S(O)ₚ, wherein said chain is optionally, substituted by one or more groups independently selected from oxo, halogen, an aryl group, a heteroaryl group or a heterocyclyl group, it will be clear to persons skilled in the art that the heteroatom may replace a primary, secondary or tertiary carbon, that is CH₃, -CH₂- or a -CH- or a branched carbon group, as technically appropriate.

In one embodiment of the disclosure there is provided compounds of formula (I), wherein R¹ is methyl, ethyl, propyl, *iso*-propyl, butyl or *tert*-butyl, in particular ethyl, *iso*-propyl or *tert*-butyl such as *tert*-butyl*.*

In one embodiment R¹ is -C(CH₃)₂CH₂OH.

In one embodiment R² is methyl, ethyl, n-propyl, *iso*-propyl, n-butyl or *tert*-butyl, in particular methyl.

In one embodiment R² is -CH₂OH.

In one embodiment R² is in the 2, 3, or 4 position (i.e. *ortho*, *meta* or *para* position), in particular the *para* (4) position.

In one embodiment Ar is substituted with 1 or 2 groups.

In one embodiment Ar is naphthyl.

In one embodiment Ar is not substituted with optional substituents.

In one embodiment Ar is substituted with 1 or 2 groups.

In one embodiment Ar is phenyl optionally substituted by 1 or 2 substituents independently selected from C₁₋₃ alkyl or C₁₋₃ alkoxy, for example tolyl, xylyl, anisoyl, dimethoxybenzene or methoxy-methylbenzene. The phenyl ring may, for example, be linked to the nitrogen of the urea through carbon 1 and to the group L through carbon 4. In such a case the optional one or two substituents selected from C₁₋₃ alkyl or C₁₋₃ alkoxy may be located in any of the unoccupied positions in the aromatic ring, for example in position 2 or in position 3 or in positions 2 and 3 or in positions 2 and 6 or in positions 3 and 5. Embodiments encompassing other possible regioisomers also form an aspect of the present disclosure.

In one embodiment R³ is H.

In one embodiment R³ is methyl, ethyl, *n*-propyl or *iso*-propyl.

In one embodiment p is 0 or 2.

In one embodiment X is selected from, pyrrole, oxazole, thiazole, isothiazole, imidazole, pyrazole, isoxazole, oxadiazole, pyridazine, pyrimidine, pyrazine, or 1,2,3 and 1,2,4 triazole, such as pyrazole, isoxazole, oxadiazole, pyridine, pyridazine, pyrimidine, pyrazine, or 1,2,3 and 1,2,4 triazole, in particular, pyrimidine, imidazole or pyridine, and especially pyridine or pyrimidine, more specifically pyridine.

In one embodiment 1, 2, 3 or 4 carbon atoms are replaced in the alkyl chain of Q by heteroatoms independently selected from O, N, S(O)ₚ.

In one embodiment the heteroatom(s) replacing carbon(s) in the alkyl chain fragment of Q are selected from N and O.

In one embodiment Q is a saturated or unsaturated, branched or unbranched C₁₋₈ alkyl chain or a C₁₋₆ alkyl chain, wherein at least one carbon is replaced by a heteroatom selected from -O, -N, S(O)ₚ. Alternatively, in this embodiment the alkyl chain may be a C₂₋₈ alkyl or a C₃₋₆ alkyl group, such as a C₄ alkyl or a C₅ alkyl group.

In one embodiment a nitrogen atom in the alkyl chain is directly bonded to the carbonyl of the fragment -NR³C(O) and additionally may, for example, be a terminal amino group.

In one embodiment Q represents C₁₋₆ alkylNH₂ or NH₂.
In one embodiment Q represents -NHC₁₋₆ alkyl such as -NHCH₃ or -NHCH₂CH₃ or -NHCH(CH₃)₂.

In one embodiment the fragment Q is a saturated or unsaturated, branched or unbranched C₁₋₁₀ alkyl chain wherein at least one carbon (for example 1, 2, 3 or 4 carbons, in particular 1 or 2 carbons) is replaced by a heteroatom selected from O, N, S(O)ₚ, for example in such a manner as to provide a stable N-acyl group, NR³C(O)Q, wherein said chain is optionally substituted by one or more groups selected from oxo, halogen, an aryl group, a heteroaryl group or a heterocyclyl group, each aryl, heteroaryl or heterocyclyl group bearing 0 to 3 substituents independently selected from a relevant substituent listed above for compounds of formula (I), for example halogen, C₁₋₆ alkyl, C₁₋₆ alkoxy, C₁₋₆ haloalkyl, amino, C₁₋₄ mono or di-alkyl amino and C₁₋₄ mono or di-acyl amino.

In one embodiment the latter chain is optionally substituted by one or more groups selected from oxo, halogen, an aryl group, a heteroaryl group or a heterocyclyl group, each aryl, heteroaryl or heterocyclyl group bearing 0 to 3 substituents selected from halogen, C₁₋₆ alkyl, C₁₋₆ alkoxy, C₁₋₆ haloalkyl, amino, and C₁₋₄ mono or di-alkyl amino.

In one embodiment Q is C₁₋₄alkyl-V-R⁴, such as C₁₋₃alkyl-V-R⁴ wherein:
V is a heteroatom selected from NR^{V}, O or S(O)ₚ;
R^{V} represents H or C₁₋₃ alkyl;
R⁴ is H or -C₁₋₃alkyl, and p is as defined above,
with the proviso that the total alkyl chain length is not more than 10 carbon atoms, including replacement heteroatoms and that the resulting radical Q is a stable group, for example - CH₂SCH₃, -CH₂SO₂CH₃, -CH₂NHCH₃, -CH₂N(CH₃)₂, -C(CH₃)₂NHCH₃, -CH(CH₃)N(CH₃)₂, - (CH₂)₃CHNHCH₃, -(CH₂)₃N(CH₃)₂, -CH₂OH, -CH₂OCH₃, -CH(CH₃)OCH₃, or -(CH₂)₂OCH₃.

In one embodiment Q is C₁₋₃ alkyl-V-(C₁₋₃ alkyl-Z-R⁵)ₖ such as C₁₋₃ alkyl-V-(C₂₋₃ alkyl-Z-R⁵)ₖ wherein:
V is a heteroatom selected from N, NH, O or S(O)ₚ, such as N or NH
(V will be selected from N in the case where k = 2, or NH, O or S(O)ₚ, in the case where k =1, in particular NH);
Z is independently selected from NH, O or S(O)ₚ;
R⁵ is H or -C₁₋₃alkyl;
k is an integer 1 or 2 (such as 1); and
p is as defined above,
with the proviso that the total alkyl chain length is not more than 10 carbon atoms, including replacement heteroatoms and that the resulting radical Q is a stable group. Suitably Q is C₁₋₃alkyl-V-C₁₋₃alkyl-OCH₃ for example C₁₋₃alkyl-V-C₂₋₃alkyl-OCH₃ such as C₁₋₃alkyl-V-(CH₂)₂OCH₃, in particular -CH₂O(CH₂)₂OCH₃ and CH₂S(CH₂)₂OCH₃, or -CH₂NH(CH₂)₂OCH₃, C₁₋₃alkyl-V-(C₁₋₃alkyl-OCH₃)ₖ wherein k represents 2, for example C₁₋₃alkyl-V-(C₂₋₃alkyl-OCH₃)ₖ such as -CH₂N[(CH₂)₂OCH₃]₂.

In one embodiment Q is C₁₋₃ alkyl-V-C₁₋₂ alkyl-Z-C₁₋₂ alkyl-Y-R⁶, or C₁₋₃ alkyl-V-C₂₋₃ alkyl-Z-C₂₋₃ alkyl-Y-R⁶, wherein V, Z and Y are independently a heteroatom selected from NH, O or S(O)ₚ,
R⁶ is H or methyl, and
p is as defined above,
with the proviso that the total alkyl chain length is not more than 10 carbon atoms, including replacement heteroatoms and that the resulting radical Q is a stable group. Suitably Q is - CH₂V(CH₂)₂O(CH₂)₂OCH₃, such as -CH₂O(CH₂)₂O(CH₂)₂OCH₃, -CH₂NH(CH₂)₂O(CH₂)₂OCH₃, or -CH₂S(CH₂)₂O(CH₂)₂OCH₃.

In one embodiment Q represents -NR⁷R⁸ and -NR³C(O)Q forms a urea, where R⁷ and R⁸ independently represent hydrogen or a C₁₋₉ saturated or unsaturated, branched or unbranched alkyl chain, wherein one or more carbons, such as 1, 2 or 3 are optionally replaced by a heteroatom selected from O, N or S(O)ₚ. Said chain is optionally substituted by one or more groups independently selected from oxo, halogen, an aryl group, a heteroaryl group, a heterocyclyl or C₃₋₈ cycloalkyl group, each aryl, heteroaryl or heterocyclyl group bearing 0 to 3 substituents independently selected from the relevant substituents listed above for compounds of formula (I), for example halogen, C₁₋₆ alkyl, C₁₋₆ alkoxy, C₁₋₆ haloalkyl, amino, C₁₋₄ mono or di-alkyl amino and C₁₋₄ mono or di-acyl amino with the proviso that the total alkyl chain length is not more than 10 carbon atoms, including replacement heteroatoms and that the resulting radical Q is a stable group.

In one embodiment Q represents -NR⁷R⁸ and -NR³C(O)Q forms a urea, where R⁷ and R⁸ independently represent hydrogen or a C₁₋₉ saturated or unsaturated, branched or unbranched alkyl chain, wherein one or more carbons, such as 1, 2 or 3 are optionally replaced by a heteroatom selected from O, N or S(O)ₚ. Said chain is optionally substituted by one or more groups independently selected from oxo, halogen, an aryl group, a heteroaryl group or a heterocyclyl group, each aryl, heteroaryl or heterocyclyl group bearing 0 to 3 substituents independently selected from the relevant substituents listed above for compounds of formula (I), for example halogen, C₁₋₆ alkyl, C₁₋₆ alkoxy, C₁₋₆ haloalkyl, amino, C₁₋₄ mono or di-alkyl amino and C₁₋₄ mono or di-acyl amino with the proviso that the total alkyl chain length is not more than 10 carbon atoms, including replacement heteroatoms and that the resulting radical Q is a stable group.

In this urea embodiment in one sub-embodiment R⁷ represents hydrogen.

Examples of ureas include those in which R⁷ and R⁸ are both hydrogen and Q is - NH₂, or where Q is -NHCH₃ or -N(CH₃)₂ to provide, for example, a fragment -NR³C(O)NH₂ or -NR³C(O)NHCH₃ or -NR³C(O)N(CH₃)₂.

Examples of ureas containing a heteroatom in the alkyl chain include those in which Q is: -NH(CH₂)₂OCH₃ or -N[(CH₂)₂OCH₃)]₂. In one embodiment Q represents -NHC₂₋₆alkylOC₁₋₃alkyl, such as -NHCH₂CH₂OCH₃.

Examples of ureas containing an oxo substituent include those in which Q is -NHCH₂C(O)NH-C₂₋₃alkyl-X¹-C₁₋₃ alkyl, wherein X¹ is a heteroatom selected from N, O or S(O)ₚ and p is defined as above. Examples of the latter include those wherein Q is - NHCH₂C(O)NHCH₂CH₂OCH₃. Thus in one embodiment Q represents -NHC₁₋₄ alkylC(O)NHC₂alkylOCH₃ such as -NHCH₂C(O)NHCH₂CH₂OCH₃.

In one embodiment Q represents -NHC₁₋₄alkylC(O)R^{Q} wherein R^{Q} is selected from OH or -NR'R" where R' is hydrogen or C₁₋₃ alkyl and R" is hydrogen or C₁₋₃ alkyl, for example -NHCH₂C(O)OH, -NHCH₂C(O)NH₂ or -NHCH₂C(O)NHCH₃ such as -NHCH₂C(O)OH or - NHCH₂C(O)NHCH₃.

In one embodiment Q represents -NHC₁₋₄ alkylC(O)OC₁₋₃alkyl,such as -NHCH₂C(O)OCH₂CH₃.

In a further urea sub-embodiment Q represents -N-R⁹C₁₋₃ alkyl-V-(C₁₋₃ alkyl-Z-R¹⁰)ₖ for example -N-R⁹C₂₋₃ alkyl-V-(C₂₋₃alkyl-Z-R¹⁰)ₖ wherein:
V represents N, NH, O, S(O)ₚ;
Z represents NH, O, S(O)ₚ;
k is an integer 1 or 2;
p is an integer 0, 1 or 2
R⁹ represents H or C₁₋₃ alkyl-V-(C₁₋₃ alkyl-Z-R¹⁰)ₖ such as C₂₋₃ alkyl-V-(C₂₋₃ alkyl-Z-R¹⁰)ₖ; and
R¹⁰ is H or C₁₋₃ alkyl such as C₁₋₃ alkyl;
with the proviso that the total alkyl chain length is not more than 10 carbon atoms, including replacement heteroatoms and that the resulting radical Q is a stable group.

In one embodiment Q is a saturated or unsaturated, branched or unbranched C₁₋₁₀ alkyl chain, wherein at least one carbon is replaced by a heteroatom selected from O, N, and S(O)ₚ, wherein said chain is substituted by an aryl group bearing 0 to 3 substituents, for example 1, 2 or 3, such as 1 or 2 substituents independently selected from the relevant substituents listed above for compounds of formula (I), for example from halogen, C₁₋₆ alkyl, C₁₋₆ alkoxy, C₁₋₆ haloalkyl, amino and C₁₋₄ mono or di-alkyl amino and C₁₋₄ mono or di-acyl amino, such as a saturated or unsaturated, branched or unbranched C₁₋₁₀ alkyl chain, wherein at least one carbon is replaced by a heteroatom selected from O, N, and S(O)ₚ, wherein said chain is substituted by an aryl group bearing 0 to 3 substituents, for example 1, 2 or 3, such as 1 or 2 substituents independently selected from halogen, C₁₋₆ alkyl, C₁₋₆ alkoxy, C₁₋₆ haloalkyl, amino and C₁₋₄ mono or di-alkyl amino. In one embodiment the said aryl group is phenyl, for example substituted phenyl or unsubstituted phenyl.

In one embodiment Q represents -NHC₀₋₆ alkylphenyl, such as -NHphenyl or NHbenzyl.

Examples of the fragment -NR³C(O)Q wherein Q comprises substituted benzyl include:
-NR³C(O)CH₂NHCH₂C₆H₄(OCH₃) such as -NHC(O)CH₂NHCH₂C₆H₄(OCH₃), for example where the methoxy substituent is in the *ortho*, *meta* or *para* position*,* such as the *para* position.

In one embodiment Q is a saturated or unsaturated, branched or unbranched C₁₋₁₀ alkyl chain, wherein at least one carbon is replaced by a heteroatom selected from O, N, and S(O)ₚ, wherein said chain is substituted by a heteroaryl group bearing 0 to 3 substituents (for example 1, 2 or 3, such as 1 or 2 substituents) independently selected from the relevant substituents listed above for compounds of formula (I), for example halogen, C₁₋₆ alkyl, C₁₋₆ alkoxy, C₁₋₆ alkyl amino, C₁₋₄ mono or di-alkyl amino and C₁₋₄ mono or di-acyl amino, such as a saturated or unsaturated, branched or unbranched C₁₋₁₀ alkyl chain, wherein at least one carbon is replaced by a heteroatom selected from O, N, and S(O)ₚ, wherein said chain is substituted by a heteroaryl group bearing 0 to 3 substituents for example 1, 2 or 3, such as 1 or 2 substituents selected from halogen, C₁₋₆ alkyl, C₁₋₆ alkoxy, C₁₋₆ alkyl amino, C₁₋₄ mono or di-alkyl amino. In one embodiment the said heteroaryl group is selected from, thiophene, oxazole, thiazole, isothiazole, imidazole, pyrazole, isoxazole, isothiazole, oxadiazole, 1,2,3 or 1,2,4 triazole, pyridine, pyridazine, pyrimidine, pyrazine and, in particular pyridine and pyrimidine, especially pyridine.

In one embodiment Q represents -NHC₁₋₆ alkylheteroaryl, for example -NH(CH₂)₃imidazolyl or -NHCH₂isoxazole wherein the isoxazole is optionally substituted such as -NHCH₂isoxazole(CH₃).

In one embodiment Q represents -NHC₁₋₄ alkylC(O)NHC₁₋₃alkylheteroaryl, for example a nitrogen containing heteroaryl group or a nitrogen and oxygen containing heteroaryl, more specifically -NHCH₂C(O)NHCH₂CH₂pyridinyl, in particular where pyridinyl is linked through carbon, for example pyridin-4-yl or -NHCH₂C(O)NHCH₂CH₂CH₂imidazolyl, in particular where imidazolyl is linked through nitrogen.

In one embodiment Q is a saturated or unsaturated, branched or unbranched C₁₋₁₀ alkyl chain, wherein at least one carbon is replaced by a heteroatom selected from O, N and S(O)ₚ wherein said chain is substituted by a heterocyclyl group bearing 0 to 3 substituents (for example 1, 2 or 3, such as 1 or 2 substituents) independently selected from the relevant substituents listed above for compounds of formula (I), for example halogen, C₁₋₆ alkyl, C₁₋₆ alkoxy, C₁₋₆ haloalkyl amino, C₁₋₄ mono or di-alkyl amino and C₁₋₄ mono or di-acyl amino, such as a saturated or unsaturated, branched or unbranched C₁₋₁₀ alkyl chain, wherein at least one carbon is replaced by a heteroatom selected from O, N and S(O)ₚ wherein said chain is substituted by a heterocyclyl group bearing 0 to 3 substituents, for example 1, 2 or 3, such as 1 or 2 substituents selected from halogen, C₁₋₆ alkyl, C₁₋₆ alkoxy, C₁₋₆ haloalkyl amino, C₁₋₄ mono or di-alkyl amino.

In one embodiment said heterocyclyl is selected, from a 5 or 6 membered saturated or partially unsaturated ring system comprising one or more (for example 1, 2 or 3 in particular 1 or 2) heteroatoms independently selected from O, N and S, for example pyrrolidine, tetrahydrofuran, tetrahydrothiophene, piperidine, piperazine, morpholine, 1,4-dioxane, pyrrolidine and oxoimidazolidine such as pyrrolidine, tetrahydrofuran, tetrahydrothiophene, piperidine, piperazine, morpholine, and 1,4-dioxane, in particular piperidine, piperazine, and morpholine.

A heterocyclic group may be linked to the alkyl chain of Q or to the carbonyl of -NR³C(O)- through carbon or nitrogen, in particular a nitrogen atom.

In one embodiment Q is -C₀₋₃alkylheterocycle (for example-C₀₋₁alkylheterocycle) said heterocyclyl group comprising at least one heteroatom (for example 1, 2 or 3, in particular 1 or 2, heteroatoms) selected from O, N and S, and is optionally substituted by one or two or three groups independently selected from the relevant substituents listed above for compounds of formula (I), for example halogen, C₁₋₆ alkyl, C₁₋₆ alkoxy, C₁₋₆ haloalkyl, amino, C₁₋₄ mono and di-alkyl amino and C₁₋₄ mono or di-acyl amino.

In one embodiment in which Q is -C₀alkylheterocycle, the heterocycle is linked through carbon, and is, for example, a C-linked tetrahydropyran or a C-linked piperidine or a C-linked morpholine or a C-linked piperazine.

In one embodiment in which Q is -C₀alkylheterocycle, the heterocyclic group containing one or more N atoms is linked through N. This embodiment provides for ureas in which one of the urea nitrogens is embedded within a heterocyclic ring. Examples of this embodiment include, but are not limited to, an *N*-linked morpholine or an *N*-linked piperidine or an *N*-linked piperazine, said *N*-linked piperizinyl group optionally bearing an additional Cor N- substituent (such as an *N*-methyl group or *N*-CH₂CH₂OCH₃ group . In one embodiment Q is a heterocyclyl linked through nitrogen such as piperidinyl, in particular 4-hydroxypiperidinyl or piperazinyl, such as 4-methyl piperazinyl.

In one embodiment Q represents a heterocyclyl group, for example a nitrogen containing heterocyclyl group, in particular linked through N, such as morpholinyl or piperazinyl optionally substituted by methyl, especially 4-methyl, or piperizinyl.

In one embodiment Q is a -C₁alkylheterocycle, for example tetrahydropyranylmethyl or a *C*- or *N*-linked piperazinylmethyl optionally bearing a substituent (for example a C₁₋₆ alkyl substituent such as methyl or a C₁₋₆ alkoxy substituent such as -CH₂CH₂OCH₃). Additional examples include a *C*- or *N*-linked pyrrolidinylmethyl, or a *C*- or *N*- linked oxoimidazolinylmethyl (such as 2-oxoimidazolidinylmethyl, said heterocycle optionally bearing a substituent (such as *N*-methyl or *N*-SO₂CH₃).

In one embodiment Q represents -NHheterocyclyl (wherein the heterocyclyl bears 0 to 3 substituents selected from the relevant list of substituents listed above for compounds of formula (I), for example halogen, hydroxy, C₁₋₆ alkyl, C₁₋₆ alkoxy, C₁₋₆ haloalkyl, amino, C₁₋₄ mono or di-alkyl amino, -S(O)_{q}C₁₋₆ alkyl, C₁₋₄ mono or di-acyl amino, C₀₋₆ alkylC(O)C₁₋₆ alkyl or C₀₋₆ alkylC(O)C₁₋₆ heteroalkyl), such as where the ring is linked through carbon, for example 2-piperidinyl or 3-piperidinyl or 4-piperidinyl, in particular 1-acetylpiperidin-4-yl, 1-methylpiperidin-4-yl, 1-(methylsulfonyl)piperidin-4-yl or 1-(2-(2-methoxyethoxy)acetyl) piperidin-4-yl

In one embodiment Q represents -NHC₁₋₆ alkylheterocyclyl for example a nitrogen containing heterocyclyl group, in particular one linked through nitrogen, such as - NHCH₂CH₂morpholine, -NH(CH₂)₃morpholine or -NH(CH₂)₄morpholine.

In one embodiment Q represents -NHC₁₋₆ alkylC(O)heterocyclyl (wherein the heterocyclyl bears 0 to 3 substituents selected from the relevant list of substituents listed above for compounds of formula (I), for example halogen, hydroxy, C₁₋₆ alkyl, C₁₋₆ alkoxy, C₁₋₆ haloalkyl, amino, C₁₋₄ mono or di-alkyl amino, C₁₋₄ mono or di-acyl amino, C₀₋₆ alkylC(O)C₁₋₆ alkyl or C₀₋₆ alkylC(O)C₁₋₆ heteroalkyl) for example a nitrogen containing heterocyclyl group, in particular one linked through nitrogen, such as -NHCH₂C(O)-1-pyrrolindinyl, -NHCH₂C(O)-1-piperidinyl, -NHCH₂C(O)-4-morpholinyl or -NHCH₂C(O)piperazinyl such as -NHCH₂C(O)-4-methyl-1-piperazinyl.

In one embodiment Q represents -NHC₁₋₄ alkylC(O)NHC₁₋₃alkylheterocyclyl for example a nitrogen containing heterocyclyl group or a nitrogen and/or oxygen containing heterocyclyl, such as -NHCH₂C(O)NHCH₂CH₂morpholinyl, in particular where morpholinyl is linked through nitrogen.

In one embodiment Q represents -N(C₁₋₃ alkyl)C₁₋₆ alkylheterocyclyl, for example a nitrogen containing heterocyclyl group, in particular linked through nitrogen, such as - N(CH₃)CH₂CH₂morpholine, -N(CH₃)(CH₂)₃morpholine or -N(CH₃)-(CH₂)₄morpholine.

In one embodiment Q is -C₁₋₃alkyl-G-C₁₋₃alkylheterocycle wherein G is a heteroatom selected from NH, O or S(O)ₚ said heterocyclyl group comprising at least one heteroatom (for example 1, 2 or 3, in particular 1 or 2, heteroatoms) selected from O, N, and S, and is optionally substituted by one or two or three groups independently selected from relevant substituents listed above for compounds of formula (I), for example halogen, C₁₋₆ alkyl, C₁₋₆ alkoxy, C₁₋₆ haloalkyl, amino, C₁₋₄ mono and di-alkyl amino and C₁₋₄ mono or di-acyl amino such as one or two or three groups halogen, C₁₋₆ alkyl, C₁₋₆ alkoxy, C₁₋₆ haloalkyl, amino, C₁₋₄ mono and di-alkyl amino. Suitably Q is -CH₂G(CH₂)₂heterocycle for example -CH₂G(CH₂)₂-tetrahydropyranyl; or -CH₂G(CH₂)₂morpholinyl in which the heterocyclyl is linked through nitrogen or carbon; or CH₂G(CH₂)₂piperazinyl in which the heterocyclyl is linked through nitrogen or carbon and optionally bearing a further C- or N- substituent (for example a C₁₋₆ alkyl substituent such as methyl or a C₁₋₆ alkoxy substituent such as -CH₂CH₂OCH₃); or - CH₂G(CH₂)₂pyrrolidinyl, in which the heterocyclyl is linked through nitrogen or carbon, for example linked through nitrogen; or -CH₂G(CH₂)₂-oxoimidazolinyl (such as 2-oxoimidazolidinyl) for example linked through N and optionally bearing an additional C- or N-substituent (such as *N*-methyl or *N*-SO₂CH₃), and in which G is O or NH.

In one embodiment G is O.

In one embodiment G is NH.

In one embodiment Q is a saturated or unsaturated C₁₋₁₀ alkyl chain wherein at least one carbon (for example 1, 2 or 3 carbons) is replaced by a heteroatom selected from O, N, S(O)ₚ wherein said chain is substituted by a C₃₋₈ carbocyclyl group and said alkyl chain is optionally substituted by one or more (for example 1 or 2) groups selected from oxo and halogen. In one embodiment said C₃₋₈ carbocyclyl group bears one or more groups (for example 1, 2 or 3 groups) independently selected from halogen, hydroxyl, C₁₋₆ alkyl, C₁₋₆ alkoxy, C₁₋₆ haloalkyl, amino, C₁₋₄ mono or di-alkyl amino, C₁₋₄ mono or di-acyl amino, S(O)_{q}C₁₋₆ alkyl, C₀₋₆ alkylC(O)C₁₋₆ alkyl or C₀₋₆ alkylC(O)C₁₋₆ heteroalkyl.

In one embodiment Q represents -NHC₃₋₆ cycloalkyl, such as -NHcyclopropyl, -NHcyclopentyl or-NHcyclohexyl.

In one embodiment the aryl, heteroaryl or heterocyclyl group bears at least one - S(O)_{q}C₁₋₆ alkyl substituent and optionally bears one or two further relevant substituents independently selected from the list of substituents defined above for compounds of formula (I).

In one embodiment the C₅₋₆ heterocycle bears at least one -S(O)_{q}C₁₋₆ alkyl substituent and optionally bears one or two further substituents independently selected from the relevant list of substituents defined above for compounds of formula (I).

In one embodiment the aryl, heteroaryl or heterocyclyl group bears at least one hydroxyl substituent and optionally bears one or two further substituents independently selected from the relevant list of substituents defined above for compounds of formula (I).

In one embodiment the C₅₋₆heterocycle bears at least one hydroxyl substituent and optionally bears one or two further substituents independently selected from the relevant list of substituents defined above for compounds of formula (I).

In one embodiment the aryl, heteroaryl or heterocyclyl group bears at least one C₁₋₄ mono and/or di-acyl amino substituent and optionally bears one or two further substituents independently selected from the relevant list defined above for compounds of formula (I).

In one embodiment the C₅₋₆heterocycle bears at least one C₁₋₄ mono and/or di-acyl amino substituent and optionally bears one or two further substituents independently selected from the relevant list defined above for compounds of formula (I).

In one embodiment the aryl, heteroaryl or heterocyclyl group bears at least one C₀₋₆ alkylC(O)C₁₋₆ heteroalkyl substituent and optionally bears one or two further substituents independently selected from the relevant list defined above for compounds of formula (I).

In one embodiment the C₅₋₆heterocycle bears at least one C₀₋₆ alkylC(O)C₁₋₆ heteroalkyl substituent and optionally bears one or two further substituents independently selected from the relevant list defined above for compounds of formula (I).

In one embodiment the aryl, heteroaryl or heterocyclyl group bears at least one C₀₋₆ alkylC(O)C₁₋₆ alkyl substituent and optionally bears one or two further substituents independently selected from the relevant list defined above for compounds of formula (I).

In one embodiment the C₅₋₆heterocycle bears at least one C₀₋₆ alkylC(O)C₁₋₆ alkyl substituent and optionally bears one or two further substituents independently selected from the relevant substituents defined above for compounds of formula (I).

In one embodiment Q represents tetrahydrofuranyl, morpholinyl, piperidinyl such as piperidinyl bearing one hydroxyl substituent, piperazinyl such as piperazinyl bearing one methyl substituent or pyrrolidinyl such a pyrrolidinyl bearing one di-methyl amino substituent. The ring may be linked through the heteroatom, such as nitrogen. Alternatively, the ring may be linked through carbon. The substituent may, for example be *para* relative to the atom through which the ring is linked to the remainder of the molecule.

In one embodiment the alkyl chain fragment of Q does not bear any optional substituents.

In one embodiment the alkyl chain is saturated.

In one embodiment the alkyl chain is unbranched.

In one embodiment the alkyl chain fragment of Q bears 1, 2, or 3, for example 1 or 2, in particular 1 optional substituent.

It will be clear to persons skilled in the art that the heteroatom may replace a primary, secondary or tertiary carbon, that is a CH₃, -CH₂- or a -CH-, group, as technically appropriate.

In one embodiment p is 0 or 2.

In one embodiment p is 1.

In one embodiment compounds of the disclosure include those in which the fragment Q is:
-CH₂OH;
-CH₂OC₁₋₆ alkyl, in particular -CH₂OCH₃;
-CH₂CH₂OCH₃;
-CH₂O(CH₂)₂OCH₃;
-CH(CH₃)OCH₃;
-CH₂NHCH₃ or -CH₂N(CH₃)₂
-CH₂NHCH₂CH₂OCH₃ or -CH₂NHC(O)CH₂OCH₃;
-CH₂SCH₃, -CH₂S(O)₂CH₃ or-CH₂NHC(O)CH₂S(O)₂CH₃; or
-CH₂NHC(O)CH₂.

In one embodiment compounds of the disclosure include those in which the fragment -NR³C(O)Q in formula (I) is represented by:
-NR³C(O)CH₂OH, in particular -NHC(O)CH₂OH;
-NR³C(O)CH₂OC₁₋₆ alkyl, in particular -NR³C(O)CH₂OCH₃, especially
-NHC(O)CH₂OCH₃;
-NR³C(O)CH₂O(CH₂)₂OCH₃, in particular-NHC(O)CH₂O(CH₂)₂OCH₃;
-NR³C(O)CH(CH₃)OCH₃ in particular-NHC(O)CH(CH₃)OCH₃;
-NR³C(O)CH(CH₃)NHC₁₋₃alkyl in particular -NHC(O)CH(CH₃)NHCH₃;
-NR³C(O)CH(CH₃)N(C₁₋₃alkyl)₂ in particular -NHC(O)CH(CH₃)N(CH₃)₂;
-NR³C(O)C(CH₃)₂NHCH₃ in particular -NHC(O)C(CH₃)₂NHCH₃;
-NR³C(O)(CH₂)₂OC₁₋₆alkyl, such as -NR³C(O)(CH₂)₂OCH₃, in particular
-NHC(O)(CH₂)₂OCH₃;
-NR³C(O)(CH₂)₃NHC₁₋₃alkyl in particular -NHC(O)(CH₂)₃NHCH₃;
-NR³C(O)(CH₂)₃N(C₁₋₃alkyl)₂ in particular -NHC(O)(CH₂)₃N(CH₃)₂;
-NR³C(O)CH₂NHC₁₋₃alkyl in particular -NHC(O)CH₂NHCH₃;
-NR³C(O)CH₂NH(CH₂)₂OCH₃ in particular -NHC(O)CH₂NH(CH₂)₂OCH₃;
-NR³C(O)CH₂SCH₃, in particular -NHC(O)CH₂SCH₃;
-NR³C(O)CH₂S(CH₂)₂OCH₃, in particular -NHC(O)CH₂S(CH₂)₂OCH₃;
-NR³C(O)CH₂S(CH₂)₂O(CH₂)₂OCH₃, in particular -NHC(O)CH₂S(CH₂)₂O(CH₂)₂OCH₃
-NR³C(O)CH₂SOCH₃, in particular -NHC(O)CH₂SOCH₃
-NR³C(O)CH₂S(O)₂CH₃, in particular -NHC(O)CH₂S(O)₂CH₃;
-NR³C(O)CH₂N[(CH₂)₂OCH₃]₂ in particular -NHC(O)CH₂N[(CH₂)₂OCH₃]₂;
-NR³C(O)NH₂ in particular -NHC(O)NH₂;
-NR³C(O)NHC₁₋₉ alkyl, such as NR³C(O)NHC₁₋₇ alkyl, in particular -NHC(O)NHCH₃
-NR³C(O)N(C₁₋₄ alkyl)C₁₋₅ alkyl in particular -NHC(O)N(CH₃)₂; or
-NR³C(O)NHCH₂CONH(CH₂)₂OCH₃ in particular -NHC(O)NHCH₂CONH(CH₂)₂OCH₃.

In one embodiment compounds of the disclosure include compounds of formula (I) in which the fragment -NR³C(O)C₀₋₈alkylheterocyclyl is represented by:
-NHC(O)-(tetrahydropyranyl), such as -NHC(O)-(tetrahydro-2*H*-pyran-4-yl):
   -NHC(O)-(morpholinyl) such as -NHC(O)-(4-morpholinyl) or -NHC(O)-(3-morpholinyl);
   -NHC(O)-(pyrrolidinyl), such as -NHC(O)-(pyrrolidin-1-yl);
   -NHC(O)-(piperazinyl), such as -NHC(O)-(piperazin-1-yl);
   -NHC(O)-(methylpiperazinyl), such as -NHC(O)-(4-methylpiperazin-1-yl);
   -NHC(O)-[(methoxyethyl)piperazinyl], such as -NHC(O)-[4-(2-methoxyethyl)piperazin-1-yl];
   -NHC(O)-(oxoimidazolidinyl) such as -NHC(O)-(2-oxoimidazolidinyl), in particular -NHC(O)-(2-oxoimidazolidin-1-yl);
   -NHC(O)CH₂-(tetrahydropyranyl), such as -NHC(O)CH₂-(tetrahydro-2*H*-pyran-4-yl);
   -NHC(O)CH₂-(morpholinyl), such as -NHC(O)CH₂-(4-morpholinyl);
   -NHC(O)CH₂-(pyrrolidinyl), such as -NHC(O)CH₂-(pyrrolidin-1-yl);
   -NHC(O)CH₂-(piperazinyl), such as -NHC(O)CH₂-(piperazin-1-yl);
   -NHC(O)CH₂-(methylpiperazinyl), such as -NHC(O)CH₂-(4-methylpiperazin-1-yl);
   -NHC(O)CH₂-[(methoxyethyl)piperazinyl], such as -NHC(O)CH₂-[4-(2-methoxyethyl) piperazin-1-yl];
   -NHC(O)CH₂SCH₂CH₂-(morpholinyl), such as -NHC(O)CH₂SCH₂CH₂-(4-morpholinyl), or - NHC(O)CH₂SCH₂CH₂-(3-morpholinyl); and
   -NHC(O)CH₂SO₂CH₂CH₂-(morpholinyl), such as -NHC(O)CH₂SO₂CH₂CH₂-(4-morpholinyl), or -NHC(O)CH₂SO₂CH₂CH₂-(3-morpholinyl).

In one embodiment compounds of the disclosure include compounds of formula (I) in which Q is:
-(tetrahydropyranyl), such as -(tetrahydro-2H-pyran-4-yl);
-(morpholinyl) such as -(4-morpholinyl);
-(pyrrolidinyl), such as -(pyrrolidin-1-yl);
-(piperazinyl), such as -(piperazin-1-yl);
-(methylpiperazinyl), such as -(4-methylpiperazin-1-yl);
-(methoxyethyl)piperazinyl, such as -4-(2-methoxyethyl)piperazin-1-yl;
-CH₂-(tetrahydropyranyl), such as -CH₂-(tetrahydro-2*H*-pyran-4-yl);
-CH₂-(morpholinyl), such as -CH₂-(4-morpholinyl);
-CH₂-(pyrrolidinyl), such as -CH₂-(pyrrolidin-1-yl);
-CH₂-(piperazinyl), such as -CH₂-(piperazin-1-yl);
-CH₂-(methylpiperazinyl), such as -CH₂-(4-methylpiperazin-1-yl);
-CH₂-[(methoxyethyl)piperazinyl], such as -CH₂-[4-(2-methoxyethyl)piperazin-1-yl];
-CH₂NHC(O)-tetrahydrofuran such as -CH₂NHC(O)-(tetrahydro-2*H*-pyran-4-yl);
-CH₂NHC(O)-morpholinyl such as -CH₂NHC(O)-(4-morpholinyl)
-CH₂NHC(O)-(piperazinyl), such as -CH₂NHC(O)-(piperazin-1-yl); and
- CH₂NHC(O)-(methylpiperazinyl), such as -CH₂NHC(O)-(4-methylpiperazin-1-yl).

In one embodiment of the fragment Q, the saturated or unsaturated, branched or unbranched C₁₋₁₀ alkyl chain, wherein at least one carbon is replaced by a heteroatom selected from -O, -N, S(O)ₚ is selected from: -CH₂OCH₂-, -CH₂NHCH₂-, -CH₂NH- and -CH₂OCH₂CH₂-. These fragments may optionally terminate in an aryl group, a heteroaryl group a heterocyclyl group or C₃₋₈ cycloalkyl group, such as an aryl group, a heteroaryl group a heterocyclyl group as defined for fragment Q above.

In one embodiment the disclosure relates to compounds of formula (**IA**): 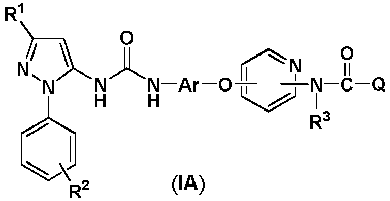 wherein R¹, R², Ar, R³ and Q are as defined above.

In a further embodiment the disclosure relates to compounds of formula (**IB**): 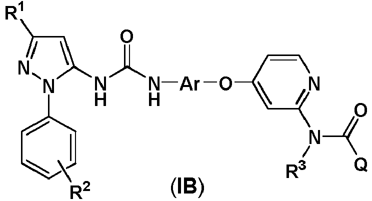 wherein R¹, R², Ar, R³ and Q are as defined above.

In yet another embodiment the disclosure relates to compounds of formula (**IC**): 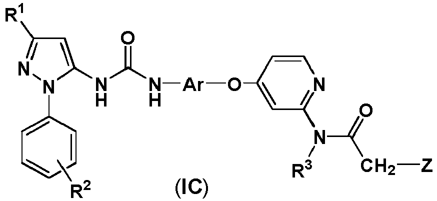
wherein R¹, R², Ar and R³ are as defined above and
Z represents a saturated or unsaturated, branched or unbranched C₁₋₉ alkyl chain, wherein at least one carbon (for example 1, 2 or 3 carbons, suitably 1 or 2, in particular 1) is replaced by a heteroatom selected from O, N, S(O)ₚ, or
a C₀₋₇ alkyl-heterocycle said heterocyclyl group comprising at least one heteroatom (for example 1, 2 or 3, suitably 1 or 2, in particular 1 heteroatom) selected from O, N and S, and is optionally substituted by one or two or three groups independently selected from the relevant substituents listed above for compounds of formula (**I**), for example halogen, C₁₋₆ alkyl, C₁₋₆ alkoxy, C₁₋₆ haloalkyl, amino, C₁₋₄ mono and di-alkyl amino.

In one embodiment of formula (**IC**) Z is -OCH₃ or -OCH₂CH₂OCH₃.

In one embodiment of formula (**IC**) Z is -SO₂CH₃.

In one embodiment of formula (**IC**) Z is -NR^{A}R^{B} wherein R^{A} and R^{B} are independently selected from hydrogen, C₁₋₆ alkyl, and C₃₋₆ alkoxy (wherein the alkoxy is not linked through oxygen) such that for example Z represents -NH₂, -NHCH₃, -N(CH₃)₂ or -NHCH₂CH₂OCH₃.

In one embodiment of formula (**IC**) Z is -S(O)ₙCH₃ wherein n is an integer 0, 1 or 2, such as 0 or 2.

In one embodiment of formula (**IC**) Z represents a 5 or 6 membered heterocycle said heterocyclyl group comprising at least one heteroatom (for example 1, 2 or 3, suitably 1 or 2, in particular 1 heteroatom) selected from O, N and S, and is optionally substituted by one or two or three groups independently selected from the relevant substituents listed above for compounds of formula (I) for example halogen, C₁₋₆ alkyl, C₁₋₆ alkoxy, C₁₋₆ haloalkyl, amino, C₁₋₄ mono and di-alkyl amino, for example:
morpholinyl (in particular linked through nitrogen) or
tetrahydropyranyl, or
piperazinyl (in particular linked through nitrogen) optionally substituted on the second nitrogen by -CH₃ or -CH₂CH₂OCH₃.

In one embodiment the disclosure relates to compounds of formula (**ID**): 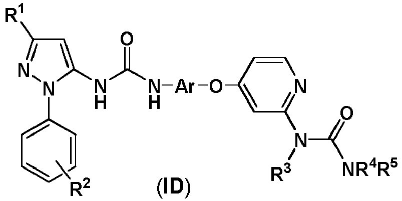
wherein R¹, R², Ar and R³ are as defined above and
R⁴ and R⁵ independently represent hydrogen, C₁₋₆ alkyl, or
R⁴ and R⁵ together with the nitrogen to which they are attached represent a 5 or 6 membered heterocycle optionally comprising a further heteroatom selected from O, N and S, wherein said heterocycle is optionally substituted by one or two or three groups independently selected from the relevant substituents listed above for compounds of formula (I), for example halogen, C₁₋₆ alkyl, C₁₋₆ alkoxy, C₁₋₆ haloalkyl, amino, C₁₋₄ mono and di-alkyl amino.

In one embodiment of compounds of formula (**ID**) the group -NR⁴R⁵ represents -NH₂, -NHCH₃ or NHCH₂CH₃.

In one embodiment of compounds of formula (**ID**) -NR⁴R⁵ represents morpholinyl or piperazinyl.

In an alternative embodiment the disclosure relates to compounds of formula (**IE**): 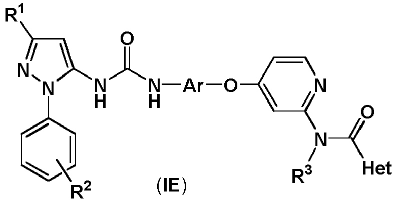
wherein R¹, R², Ar and R³ are as defined above and
Het represents a 5 or 6 membered heterocycle said heterocyclyl group comprising at least one heteroatom (for example 1, 2 or 3, suitably 1 or 2, in particular 1 heteroatom) selected from O, N and S, and is optionally substituted by one or two or three groups independently selected from the relevant substituents listed above for compounds of formula (I) for example halogen, C₁₋₆ alkyl, C₁₋₆ alkoxy, C₁₋₆ haloalkyl, amino, C₁₋₄ mono and di-alkyl amino.

In one embodiment of compounds of formula (**IE**) Het is morpholinyl or tetrahydropyranyl.
In one embodiment the compound is:
*N*-(4-(4-(3-(3-*tert*-Butyl-1-*p*-tolyl-1*H*-pyrazol-5-yl)ureido)naphthalen-1-yloxy)pyridin-2-yl)-2-(2-methoxyethoxy)acetamide;
*N*-(4-(4-(3-(3-*tert*-Butyl-1-*p*-tolyl-1*H*-pyrazol-5-yl)ureido)naphthalen-1-yloxy)pyridin-2-yl) tetrahydro-2*H*-pyran-4-carboxamide;
*N*-(4-(4-(3-(3-*tert*-Butyl-1-*p*-tolyl-1*H*-pyrazol-5-yl)ureido)naphthalen-1-yloxy)pyridin-2-yl)-2-(methylthio)acetamide;
*N*-(4-(4-(3-(3-*tert*-Butyl-1-*p*-tolyl-1*H*-pyrazol-5-yl)ureido)naphthalen-1-yloxy)pyridin-2-yl)-3-methoxypropanamide;
*N*-(4-(4-(3-(3-*tert*-Butyl-1-*p*-tolyl-1*H*-pyrazol-5-yl)ureido)naphthalen-1-yloxy)pyridin-2-yl)-2-hydroxyacetamide;
*N*-(4-(4-(3-(3-Isopropyl-1-*p*-tolyl-1*H*-pyrazol-5-yl)ureido)naphthalen-1-yloxy)pyridin-2-yl)-2-methoxyacetamide;
*N*-(4-(4-(3-(3-Ethyl-1-*p*-tolyl-1*H*-pyrazol-5-yl)ureido)naphthalen-1-yloxy)pyridin-2-yl)-2-methoxyacetamide;
N-(4-(4-(3-(3-tert-Butyl-1-p-tolyl-1 H-pyrazol-5-yl)ureido)naphthalen-1-yloxy)pyridin-2-yl)-2-methoxyacetamide;
*N*-(4-(4-(3-(3-(1-Hydroxy-2-methylpropan-2-yl)-1-*p*-tolyl-1*H*-pyrazol-5-yl)ureido)naphthalen-1-yloxy)pyridin-2-yl)-2-methoxyacetamide;
*N*-(4-(4-(3-(3-tert-Butyl-1-(2,3,5,6-tetradeutero-4-(trideuteromethyl)phenyl)-1H-pyrazol-5-yl)ureido)naphthalen-1-yloxy)pyridin-2-yl)-2-methoxyacetamide;
*N*-(4-(4-(3-(3-tert-Butyl-1-*p*-tolyl-1H-pyrazol-5-yl)ureido)naphthalen-1-yloxy)pyridin-2-yl)-2-morpholinoacetamide;
*N*-(4-(4-(3-(3-*tert*-Butyl-1-*p*-tolyl-1*H*-pyrazol-5-yl)ureido)naphthalen-1-yloxy)pyridin-2-yl)-(dimethylamino)acetamide;
*N-*(4-(4-(3-(3-*tert*-Butyl-1-*p*-tolyl-1*H*-pyrazol-5-yl)ureido)naphthalen-1-yloxy)pyridin-2-yl)-2-(2-methoxyethylamino)acetamide;
*N*-(4-(4-(3-(3-*tert*-Butyl-1-*p*-tolyl-1*H*-pyrazol-5-yl)ureido)naphthalen-1-yloxy)pyridin-2-yl)-2-ureidoacetamide;
*N*-(4-(4-(3-(3-*tert*-Butyl-1-*p*-tolyl-1*H*-pyrazol-5-yl)ureido)naphthalen-1-yloxy)pyridin-2-yl)-2-(2-methoxyacetamido)acetamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)tetrahydro-2H-pyran-4-carboxamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)isonicotinamide;
*N*-(4-(4-(3-(3-*tert*-Butyl-1-*p*-tolyl-1*H*-pyrazol-5-yl)ureido)naphthalen-1-yloxy)pyridin-2-yl)-2-(2-(methylsulfonyl)acetamido)acetamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)-3-morpholinopropanamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)morpholine-4-carboxamide;
*N*-(2-(4-(4-(3-(3-*tert*-Butyl-1-*p*-tolyl-1*H*-pyrazol-5-yl)ureido)naphthalen-1-yloxy)pyridin-2-ylamino)-2-oxoethyl)-2,6-difluoro-3-(2-(2-methoxyethoxy)ethoxy)benzamide;
*N*-(4-(4-(3-(3-*tert*-Butyl-1-*p*-tolyl-1*H*-pyrazol-5-yl)ureido)phenoxy)pyridin-2-yl)-2-methoxy acetamide;
*N*-(4-(4-(3-(3-*tert*-Butyl-1-*p*-tolyl-1*H*-pyrazol-5-yl)ureido)-2-methylphenoxy)pyridin-2-yl)-2-methoxyacetamide;
*N*-(4-(4-(3-(3-*tert*-Butyl-1-*p*-tolyl-1*H*-pyrazol-5-yl)ureido)-3-methylphenoxy)pyridin-2-yl)-2-methoxyacetamide;
*N*-(4-(4-(3-(3-*tert*-Butyl-1-*p*-tolyl-1*H*-pyrazol-5-yl)ureido)-2-methoxyphenoxy)pyridin-2-yl)-2-methoxyacetamide;
*N*-(4-(4-(3-(3-*tert*-Butyl-1-*p*-tolyl-1*H*-pyrazol-5-yl)ureido)-2,3-dimethylphenoxy)pyridin-2-yl)-2-methoxyacetamide;
*N*-(4-(4-(3-(3-*tert*-Butyl-1-*p*-tolyl-1*H*-pyrazol-5-yl)ureido)-3-methoxyphenoxy)pyridin-2-yl)-2-methoxyacetamide;
*N*-Ethyl-N'-4-(4-(3-(3-*tert*-butyl-1-*p*-tolyl-1*H*-pyrazol-5-yl)ureido) naphthalen-1-yloxy)pyridin-2-ylurea;
4-(4-(3-(3-*tert*-Butyl-1-*p*-tolyl-1*H*-pyrazol-5-yl)ureido)naphthalen-1-yloxy)pyridin-2-ylurea;
*N*-Propan-2-yl-*N'*-4-(4-(3-(3-*tert*-butyl-1-*p*-tolyl-1*H*-pyrazol-5-yl)ureido)naphthalen-1-yloxy) pyridin-2-ylurea;
1-(3-(*tert*-Butyl)-1-(*p*-tolyl)-1*H*-pyrazol-5-yl)-3-(4-((2-(3-phenylureido)pyridin-4-yl)oxy) naphthalen-1-yl)urea;
1-(4-((2-(3-Benzylureido)pyridin-4-yl)oxy)naphthalen-1-yl)-3-(3-(*tert*-butyl)-1-(*p*-tolyl)-1*H-*pyrazol-5-yl)urea;
1-(4-((2-(3-Cyclopropylureido)pyridin-4-yl)oxy)naphthalen-1-yl)-3-(3-(*tert*-butyl)-1-(*p*-tolyl)-1*H-*pyrazol-5-yl)urea;
1-(3-(*tert*-Butyl)-1-(*p*-tolyl)-1*H*-pyrazol-5-yl)-3-(4-((2-(3-(2-methoxyethyl)ureido)pyridin-4-yl) oxy)naphthalen-1-yl)urea;
1-(3-(*tert*-Butyl)-1-(*p*-tolyl)-1*H*-pyrazol-5-yl)-3-(4-((2-(3-cyclopentyl)ureido)pyridin-4-yl)oxy) naphthalen-1-yl)urea;
1-(3-(*tert*-Butyl)-1-(*p*-tolyl)-1*H*-pyrazol-5-yl)-3-(4-((2-(3-methyl)ureido)pyridin-4-yl)oxy) naphthalen-1-yl)urea;
Ethyl 2-(3-(4-((4-(3-(3-(*tert*-butyl)-1-(*p*-tolyl)-1*H*-pyrazol-5-yl)ureido)naphthalen-1-yl)oxy) pyridin-2-yl)ureido)acetate;
4-(3-(4-(4-(3-(3-*tert*-Butyl-1-*p*-tolyl-1*H*-pyrazol-5-yl)ureido)naphthalen-1-yloxy)pyridin-2-yl)ureido)piperidine;
*N*-Acetyl 4-(3-(4-(4-(3-(3-*tert*-butyl-1-*p*-tolyl-1*H*-pyrazol-5-yl)ureido)naphthalen-1-yloxy) pyridin-2-yl)ureido)piperidine;
2-(2-Methoxyethoxy)-1-(4-(3-(4-(4-(3-(3-*tert*-butyl-1-*p-*tolyl-1*H*-pyrazol-5-yl)ureido) naphthalen-1-yloxy)pyridin-2-yl)ureido)piperidin-1-yl)ethanone;
*N*-Methylsulfonyl-4-(3-(4-(4-(3-(3-*tert*-butyl-1-*p*-tolyl-1*H*-pyrazol-5-yl)ureido)naphthalen-1-yloxy)pyridin-2-yl)ureido)piperidine;
*N*-(4-(4-(3-(3-*tert*-Butyl-1-*p*-tolyl-1*H*-pyrazol-5-yl)ureido)naphthalen-1-yloxy)pyridin-2-yl) morpholine-4-carboxamide;
*N*-(4-((4-(3-(3-(*tert*-Butyl)-1-(*p*-tolyl)-1*H*-pyrazol-5-yl)ureido)naphthalen-1-yl)oxy)pyridin-2-yl)-4-methylpiperazine-1-carboxamide;
3-(4-((4-(3-(3-(*tert*-Butyl)-1-(*p*-tolyl)-1*H*-pyrazol-5-yl)ureido)naphthalen-1-yl)oxy)pyridin-2-yl)-1,1-dimethylurea;
*N*-(4-((4-(3-(3-(*tert*-Butyl)-1-(*p*-tolyl)-1*H*-pyrazol-5-yl)ureido)naphthalen-1-yl)oxy)pyridin-2-yl) piperidine-1-carboxamide;
*N*-Methyl-*N*-(2-(morpholin-4-yl)ethyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(4-(Morpholin-4-yl)butyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(2-(Morpholin-4-yl)ethyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(3-Methylisoxazol-5-yl)methyl-*N*'-4-(4-(3-(3-*tert*-butyl-1-*p*-tolyl-1*H*-pyrazol-5-yl)ureido) naphthalen-1-yloxy)pyridin-2-ylurea;
*N*-(1-Methyl)piperidin-4-yl-*N*'-4-(4-(3-(3-*tert*-butyl-1-*p*-tolyl-1*H*-pyrazol-5-yl)ureido) naphthalen-1-yloxy)pyridin-2-ylurea;
*N*-(4-(4-(3-(3-*tert*-Butyl-1-*p*-tolyl-1*H*-pyrazol-5-yl)ureido)naphthalen-1-yloxy)pyridin-2-yl)-4-hydroxypiperidine-1-carboxamide;
*N*-(3-(Imidazol-1-yl)propyl)-*N'*-4-(4-(3-(3-*tert*-butyl-1-*p*-tolyl-1*H*-pyrazol-5-yl)ureido) naphthalen-1-yloxy)pyridin-2-ylurea;
*N*-(2-(3-(4-(4-(3-(3-*tert*-Butyl-1-*p*-tolyl-1*H*-pyrazol-5-yl)ureido)naphthalen-1-yloxy)pyridin-2-yl) ureido)acetyl)pyrrolidine;
(*R*)-*N*-(4-(4-(3-(3-*tert*-Butyl-1-*p*-tolyl-1*H*-pyrazol-5-yl)ureido)naphthalen-1-yloxy)pyridin-2-yl)-3-(dimethylamino)pyrrolidine-1-carboxamide;
*N*-(4-(4-(3-(3-*tert*-Butyl-1-*p*-tolyl-1*H*-pyrazol-5-yl)ureido)naphthalen-1-yloxy)pyridin-2-yl) pyrrolidine-1-carboxamide;
2-(3-(4-(4-(3-(3-*tert*-Butyl-1-*p*-tolyl-1*H*-pyrazol-5-yl)ureido)naphthalen-1-yloxy)pyridin-2-yl) ureido)-*N*-methylacetamide;
2-(3-(4-(4-(3-(3-*tert*-Butyl-1-*p*-tolyl-1*H*-pyrazol-5-yl)ureido)naphthalen-1-yloxy)pyridin-2-yl) ureido)-*N*-(2-morpholinoethyl)acetamide;
2-(3-(4-(4-(3-(3-*tert*-Butyl-1-*p*-tolyl-1*H*-pyrazol-5-yl)ureido)naphthalen-1-yloxy)pyridin-2-yl) ureido)acetyl morpholine;
2-(3-(4-(4-(3-(3-*tert*-Butyl-1-*p*-tolyl-1*H*-pyrazol-5-yl)ureido)naphthalen-1-yloxy)pyridin-2-yl) ureido)-*N*-(2-(pyridin-4-yl)ethyl)acetamide;
*N*-(3-(1*H*-Imidazol-1-yl)propyl)-2-(3-(4-(4-(3-(3-*tert*-butyl-1-*p*-tolyl-1*H*-pyrazol-5-yl)ureido) naphthalen-1-yloxy)pyridin-2-yl)ureido)acetamide;
1-(2-(3-(4-(4-(3-(3-*tert*-Butyl-1-*p*-tolyl-1*H*-pyrazol-5-yl)ureido)naphthalen-1-yloxy)pyridin-2-yl) ureido)acetyl)-4-methylpiperazine;
*N*-(3-(1*H*-Imidazol-1-yl)propyl)-2-(3-(4-(4-(3-(3-*tert*-butyl-1-*p*-tolyl-1*H*-pyrazol-5-yl)ureido) naphthalen-1-yloxy)pyridin-2-yl)ureido)acetamide;
*N*-(6-(4-(3-(3-*tert*-Butyl-1-*p*-tolyl-1*H*-pyrazol-5-yl)ureido)naphthalen-1-yloxy)pyrimidin-4-yl)-2-methoxyacetamide;
*N*-(6-(4-(3-(3-*tert*-Butyl-1-*p*-tolyl-1*H*-pyrazol-5-yl)ureido)phenoxy)pyrimidin-4-yl)-2-methoxy acetamide;
*N*-(4-(4-(3-(3-*tert*-Butyl-1-*p*-tolyl-1*H*-pyrazol-5-yl)ureido)naphthalen-1-yloxy)pyrimidin-2-yl)-2-methoxyacetamide;
3-(4-(4-(3-(3-*tert*-Butyl-1-*p*-tolyl-1*H*-pyrazol-5-yl)ureido)naphthalen-1-yloxy)pyrimidin-2-yl) urea;
1-Methyl-3-(4-(4-(3-(3-*tert*-butyl-1-*p*-tolyl-1*H*-pyrazol-5-yl)ureido)naphthalen-1-yloxy) pyrimidin-2-yl)urea;
1,1-Dimethyl-3-(4-(4-(3-(3-*tert*-butyl-1-*p*-tolyl-1*H*-pyrazol-5-yl)ureido)naphthalen-1-yloxy) pyrimidin-2-yl)urea;
1-Cyclopropyl-3-(4-(4-(3-(3-*tert*-butyl-1-*p*-tolyl-1*H*-pyrazol-5-yl)ureido)naphthalen-1-yloxy) pyrimidin-2-yl)urea;
(4-(4-(3-(3-*tert*-Butyl-1-*p*-tolyl-1*H*-pyrazol-5-yl)ureido)naphthalen-1-yloxy)pyrimidin-2-yl) morpholine-4-carboxamide;
3-(6-(4-(3-(3-*tert*-Butyl-1-*p*-tolyl-1*H*-pyrazol-5-yl)ureido)naphthalen-1-yloxy)pyrimidin-4-yl) urea;
2-(3-(4-(4-(3-(3-*tert*-Butyl-1-*p*-tolyl-1H-pyrazol-5-yl)ureido)naphthalen-1-yloxy)pyridin-2-yl) ureido)acetic acid, or
a pharmaceutically acceptable salt thereof, including all stereoisomers, tautomers and isotopic derivatives thereof.

In one embodiment the compound is not *N*-(4-(4-(3-(3-*tert*-Butyl-1-*p*-tolyl-1*H*-pyrazol-5-yl)ureido)naphthalen-1-yloxy)pyridin-2-yl)-2-methoxyacetamide.

Examples of salts of compound (**I**) include all pharmaceutically acceptable salts, such as, without limitation, acid addition salts of strong mineral acids such as HCl and HBr salts and addition salts of strong organic acids such as a methansulfonic acid salt.

The disclosure herein extends to solvates of compounds of formula (**I**). Examples of solvates include hydrates.

The compounds of the disclosure include those where the atom specified is replaced by a naturally occurring or non-naturally occurring isotope. In one embodiment the isotope is a stable isotope. Thus the compounds of the disclosure include, for example deuterium containing compounds and the like.

The compounds described herein may include one or more chiral centres, and the disclosure extends to include racemates, enantiomers and stereoisomers resulting therefrom. In one embodiment one enantiomeric form is present in a substantially purified form that is substantially free of the corresponding enantiomeric form.

The disclosure also extends to all polymorphic forms of the compounds herein defined.

Unless the context indicates otherwise references to compounds of formula (**I**) herein includes references to one or more, such as all structures and compounds disclosed.

In one embodiment the disclosure comprises a composition comprising a compound of formula (**I**) and a pharmaceutically acceptable excipient (including a diluent or carrier) for use in the treatment or prevention of rhinovirus infection. The composition may optionally comprise a further pharmaceutically agent.

A compound of the disclosure may also be administered in combination with one or more other active ingredients e.g. active ingredients suitable for treating the above mentioned conditions. For example possible combinations for treatment of respiratory disorders include combinations with steroids (e.g. budesonide, beclomethasone dipropionate, fluticasone propionate, mometasone furoate, fluticasone furoate), beta agonists (e.g. terbutaline, salbutamol, salmeterol, formoterol) and/or xanthines (e.g. theophylline).

In one embodiment the compound or composition is dosed directly to the inflamed organ (topical therapy).

In topical therapy, efficacy can be achieved either by (i) ensuring that the drug has a sustained duration of action and is retained in the relevant organ to minimize the risks of systemic toxicity or (ii) producing a formulation which generates a "reservoir" of the active drug which is available to sustain the drug's desired effects. Approach (i) is exemplified by the anticholinergic drug tiotropium (Spiriva), which is administered topically to the lung as a treatment for COPD, and which has an exceptionally high affinity for its target receptor resulting in a very slow off rate and a consequent sustained duration of action.

In one aspect of the disclosure the compounds herein are particularly suitable for topical delivery, such as topical delivery to the lungs.

In one embodiment the compound or composition according to the disclosure is administered orally or parenterally.

In one embodiment the compounds are suitable for sensitizing patients to treatment with a corticosteroid.

Diluents and carriers may include those suitable for parenteral, oral, topical, mucosal and rectal administration, depending on the desired route of administration.

As mentioned above, such compositions may be prepared e.g. for parenteral, subcutaneous, intramuscular, intravenous, intra-articular or peri-articular administration, particularly in the form of liquid solutions or suspensions; for oral administration, particularly in the form of tablets or capsules; for topical e.g. pulmonary or intranasal administration, particularly in the form of powders, nasal drops or aerosols and transdermal administration; for mucosal administration e.g. to buccal, sublingual or vaginal mucosa, and for rectal administration e.g. in the form of a suppository.

The compositions may conveniently be administered in unit dosage form and may be prepared by any of the methods well-known in the pharmaceutical art, for example as described in Remington's Pharmaceutical Sciences, 17th ed., Mack Publishing Company, Easton, PA., (1985). Formulations for parenteral administration may contain as excipients sterile water or saline, alkylene glycols such as propylene glycol, polyalkylene glycols such as polyethylene glycol, oils of vegetable origin, hydrogenated naphthalenes and the like. Formulations for nasal administration may be solid and may contain excipients, for example, lactose or dextran, or may be aqueous or oily solutions for use in the form of nasal drops or metered spray. For buccal administration typical excipients include sugars, calcium stearate, magnesium stearate, pregelatinated starch, and the like.

Compositions suitable for oral administration may comprise one or more physiologically compatible carriers and/or excipients and may be in solid or liquid form. Tablets and capsules may be prepared with binding agents, for example, syrup, acacia, gelatin, sorbitol, tragacanth, or poly-vinylpyrollidone; fillers, such as lactose, sucrose, corn starch, calcium phosphate, sorbitol, or glycine; lubricants, such as magnesium stearate, talc, polyethylene glycol, or silica; and surfactants, such as sodium lauryl sulfate. Liquid compositions may contain conventional additives such as suspending agents, for example sorbitol syrup, methyl cellulose, sugar syrup, gelatin, carboxymethyl-cellulose, or edible fats; emulsifying agents such as lecithin, or acacia; vegetable oils such as almond oil, coconut oil, cod liver oil, or peanut oil; preservatives such as butylated hydroxyanisole (BHA) and butylated hydroxytoluene (BHT). Liquid compositions may be encapsulated in, for example, gelatin to provide a unit dosage form.

Solid oral dosage forms include tablets, two-piece hard shell capsules and soft elastic gelatin (SEG) capsules.

A dry shell formulation typically comprises of about 40% to 60% concentration of gelatin, about a 20% to 30% concentration of plasticizer (such as glycerin, sorbitol or propylene glycol) and about a 30% to 40% concentration of water. Other materials such as preservatives, dyes, opacifiers and flavours also may be present. The liquid fill material comprises a solid drug that has been dissolved, solubilized or dispersed (with suspending agents such as beeswax, hydrogenated castor oil or polyethylene glycol 4000) or a liquid drug in vehicles or combinations of vehicles such as mineral oil, vegetable oils, triglycerides, glycols, polyols and surface-active agents.

Suitably the compound of formula (**I**) is administered topically to the lung. Hence we provide according to the invention a pharmaceutical composition comprising a compound of the disclosure optionally in combination with one or more topically acceptable diluents or carriers. Topical administration to the lung may be achieved by use of an aerosol formulation. Aerosol formulations typically comprise the active ingredient suspended or dissolved in a suitable aerosol propellant, such as a chlorofluorocarbon (CFC) or a hydrofluorocarbon (HFC). Suitable CFC propellants include trichloromonofluoromethane (propellant 11), dichlorotetrafluoromethane (propellant 114), and dichlorodifluoromethane (propellant 12). Suitable HFC propellants include tetrafluoroethane (HFC-134a) and heptafluoropropane (HFC-227). The propellant typically comprises 40% to 99.5% e.g. 40% to 90% by weight of the total inhalation composition. The formulation may comprise excipients including cosolvents (e.g. ethanol) and surfactants (e.g. lecithin, sorbitan trioleate and the like). Aerosol formulations are packaged in canisters and a suitable dose is delivered by means of a metering valve (e.g. as supplied by Bespak, Valois or 3M).

Topical administration to the lung may also be achieved by use of a non-pressurised formulation such as an aqueous solution or suspension. This may be administered by means of a nebuliser. Topical administration to the lung may also be achieved by use of a dry-powder formulation. A dry powder formulation will contain the compound of the disclosure in finely divided form, typically with a mass mean diameter (MMAD) of 1-10 µm. The formulation will typically contain a topically acceptable diluent such as lactose, usually of large particle size e.g. a mass mean diameter (MMAD) of 100 µm or more. Example dry powder delivery systems include SPINHALER®, DISKHALER®, TURBOHALER®, DISKUS® and CLICKHALER®.

A compounds and/or compositions employed in the present disclosure may also be administered in combination with one or more other active ingredients e.g. active ingredients suitable for treating the above mentioned conditions. For example possible combinations for treatment of respiratory disorders include combinations with corticosteroids (e.g. budesonide, beclomethasone dipropionate, fluticasone propionate, mometasone furoate, fluticasone furoate), beta agonists (e.g. terbutaline, salbutamol, salmeterol, formoterol), anti-muscarinic (e.g. iprotropium bromide, Tiotropium), xanthines (e.g. theophylline), phophodiesterase inhibitors (roflumilast, cilomolast) and/or anti-viral compounds (e.g. ribavirin, pleconaril and/or rupintrivir).

Compounds and compositions according to the disclosure may also be useful in the treatment or prevention of rhinovirus infection in patients with chronic or persistent respiratory disorders including COPD (including chronic bronchitis and emphysema), asthma, paediatric asthma, cystic fibrosis, sarcoidosis, idiopathic pulmonary fibrosis, allergic rhinitis, rhinitis, sinusitis, especially asthma, chronic bronchitis and COPD.

Compounds and compositions of the disclosure may also re-sensitise the patient's condition to treatment with a corticosteroid, when the patient's condition has become refractory to the same.

The compounds and compositions of the present disclosure are likely to be useful in the treatment or prevention of rhinovirus infection in the patients with low/suppressed or compromised immunity, for example HIV and AIDS patients or those undergoing chemotherapy.

In one embodiment the compounds and compositions of the present disclosure are useful in the treatment of infants.

The compounds and compositions of the present the disclosure may result in lower morbidity and/or fewer complications in patients susceptible to rhinovirus infection.

The compounds and compositions of the invention are active against one or more strains, such as 1, 2, 3 or all known strains of rhinovirus.

Compounds and compositions of the disclosure are believed to be useful as anti-viral agents, for example in the treatment or prevention of rhinovirus infection. The compounds and compositions of the present disclosure may be suitable for the use in the treatment or prevention of said viral infection and in particular may be capable of reducing viral load and/or ameliorating symptoms after infection.

Treatment as employed herein is intended to refer to amelioration of the symptoms and/or that the duration of the infection in the patient is reduces in comparison to a patient who does not receive treatment for the infection.

In one embodiment the compound or compositions of the present disclosure when administered prophylactically reduces the likelihood of infection with rhinovirus by 5, 10, 15, 20, 25, 30, 35, 40, 45, 50, 55, 60% or more.

In a further aspect, the present invention provides use of a compound as described herein for the manufacture of a medicament for the treatment of the above mentioned conditions.

An effective amount will be an amount effective to cause reduction in symptoms of rhinovirus infection and/or the exacerbation of respiratory disorders (such as asthma, COPD, bronchitis and/or cystic fibrosis) by rhinovirus infection, for example an amount which causes a reduction in viral load, and may be determined by a skilled person by reference to the severity of the condition in the subject. Typically an amount of 0.1 to 10 µg/kg, e.g. 1 to 5 µg/kg or a dose of 7 to 700 µg, e.g. 70 to 350 µg per day will be suitable.

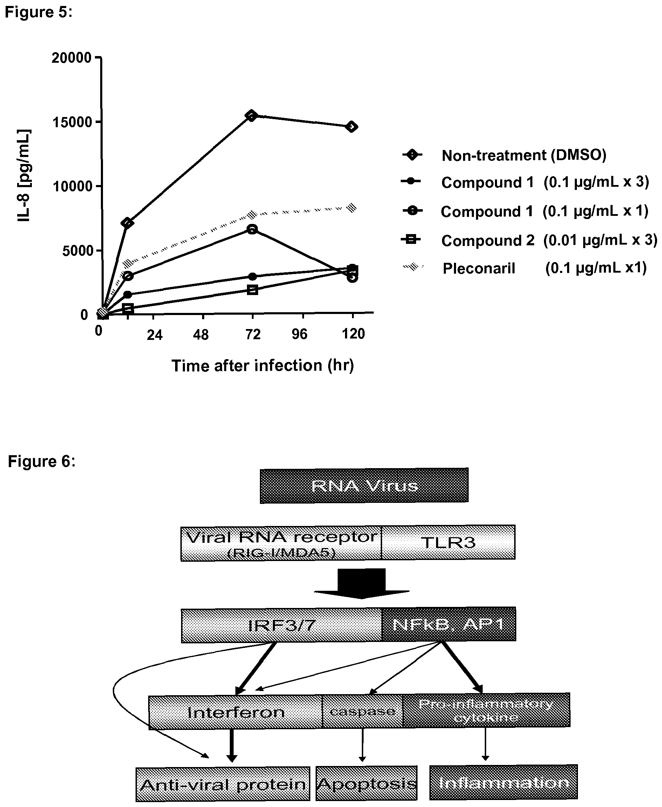

In one embodiment the disclosure herein relates to the identification for the first time of the role of IRF 3/7 and NFKB in human rhinovirus infection (**Figure 6**). As such the present invention provides for the use of a compound capable of inhibiting IRF 3/7 and/or NFKB activity for the manufacture of a medicament for the treatment or prophylaxis of infection by human rhinovirus. Furthermore, the present invention relates to compounds capable of inhibiting IRF 3/7 and/or NFKB activity identified by the methods and assays disclosed herein.

The present invention provides:
- A compound or composition capable of inhibiting c-SRC and SYK activity for use in the treatment or prophylaxis of infection by human rhinovirus (HRV), wherein the compound or composition does not comprise *N*-(4-(4-(3-(3-*tert*-Butyl-1-*p*-tolyl-1H-pyrazol-5-yl) ureido)naphthalen-1-yloxy)pyridin-2-yl)-2-methoxyacetamide; and
- Use of a compound or composition capable of inhibiting c-SRC and SYK activity for the manufacture of a medicament for the treatment or prophylaxis of infection by human rhinovirus (HRV), wherein the compound or composition does not comprise *N*-(4-(4-(3-(3-*tert*-Butyl-1-*p*-tolyl-1H-pyrazol-5-yl)ureido)naphthalen-1-yloxy)pyridin-2-yl)-2-methoxyacetamide.
In addition, there is provided a compound or use as just described wherein the compound is administered in combination with one or more anti-viral drugs such as anti-viral drugs targeting picornaviruses e.g. selected from pleconaril and analogues thereof and also a composition for separate, simultaneous or sequential use comprising a compound or composition capable of inhibiting c-SRC and SYK activity and one or more anti-viral drugs such as anti-viral drugs targeting picornaviruses e.g. selected from pleconaril and analogues thereof.

In an embodiment of the disclosure, there is provided a method for the treatment or prophylaxis of infection by rhinovirus comprising administering to a patient a therapeutically effective amount of a compound or composition capable of inhibiting c-SRC and SYK activity in a patient. An effective amount will be an amount sufficient to cause reduction in symptoms of rhinovirus infection and/or the exacerbation of respiratory disorders (such as asthma, COPD, bronchitis and/or cystic fibrosis) by rhinovirus infection, for example an amount which causes a reduction in viral load, and may be determined by a skilled person by reference to the severity of the condition in the subject and the potency of inhibition at c-SRC and SYK of the compound or composition. Typically an amount of 0.1 to 10 µg/kg, e.g. 1 to 5 µg/kg or a dose of 7 to 700 µg, e.g. 70 to 350 µg per day will be suitable.

In further embodiments of the disclosure there is provided:
- A compound, use or method as just described wherein the compound or composition is or contains a chemical inhibitor of c-SRC and SYK activity;
- A compound, use or method as just described wherein the compound or composition is or contains a biochemical inhibitor of c-SRC and SYK activity, for example the compound or composition is or contains an RNAi molecule;
- A compound, use or method as just described wherein the compound or composition is or contains a competitive inhibitor of c-SRC activity and is or contains a competitive inhibitor of SYK activity or else the compound or composition is or contains a non-competitive inhibitor of c-SRC activity and is or contains a non-competitive inhibitor of SYK activity; and

In an embodiment of the invention, there is provided:
- A method of screening for a candidate drug substance or substances intended to prevent or treat rhinovirus infection in a subject which comprises identifying one or more test substances which together or separately are capable of inhibiting c-SRC and SYK activity by measuring the effects of said test substance or substances on c-SRC and SYK activity. Specifically: such a method of screening comprises:

a. contacting c-SRC and SYK with a test substance in the presence of FRET peptide and ATP;
b. measuring the level of phosphorylation of FRET peptide after a set time period; and
c. comparing the level of phosphorylation measured to that observed in a control experiment in which c-SRC and SYK are not contacted with the test substance. The test substance may be contacted with c-SRC and SYK in the same or different assays;
or else such an method of screening comprises:
a. contacting said substance with c-SRC and SYK or cells expressing c-SRC and SYK; and
b. determining whether c-SRC and SYK enzymatic activity is inhibited;
whereby inhibition of c-SRC and SYK enzymatic activity indicates that the substance is a candidate drug substance intended to prevent or treat rhinovirus virus infection. The test substance may be contacted with c-SRC and SYK or cells expressing c-SRC and SYK in the same or different assays. The aforementioned methods of screening are, for example, *in vitro* methods.
- In the aforementioned compound, composition, use or method, suitably a single compound inhibits both c-SRC and SYK activities, but alternatively one compound inhibits c-SRC activity and another compound inhibits SYK activity.
- Suitably the inhibitory activity as measured by IC₅₀ of the compound or compounds against c-SRC is less than 1000 nM e.g. particularly less than 200 nM e.g. less than 100 nM eg less than 50 nM e.g. less than 10 nM. Suitably the inhibitory activity as measured by IC₅₀ of the compound or compounds against SYK is less than 1000 nM e.g. less than 200 nM e.g. particularly less than 100 nM eg less than 50 nM.
There is also provided:
- A compound capable of inhibiting c-SRC and SYK activity identified by an aforesaid method or compound, composition or use as aforesaid, with the proviso that it is not a compound of formula (**I**) as defined above or a pharmaceutically acceptable salt thereof.

Suitably in the compounds, compositions or uses as aforesaid the human rhinovirus (HRV) is, for example, HRV-16.

### Abbreviations

Abbreviations used herein are as defined below. Any abbreviations not defined have their generally accepted meaning.
- Ac: acetyl
- ATP: adenosine-5'-triphosphate
- Ak: protein kinase B
- aq.: aqueous
- BSA: bovine serum albumin
- cDNA: complementary deoxyribonucleic acid
- COPD: chronic obstructive pulmonary disease
- c-SRC: cellular-SRC
- DMEM: Dulbecco's modified Eagle's medium
- DMF: *N*,*N*-dimethylformamide
- DMSO: dimethyl sulfoxide
- ELISA: enzyme linked immunosorbent assay
- Et: ethyl
- EtOAc: ethyl acetate
- FCS: foetal calf serum
- FP: fluticasone propionate
- FRET: fluorescence resonance energy transfer
- GAPDH: glyceraldehyde phosphate dehydrogenase
- HCK: hemopoietic cell kinase
- Hr: hour(s)
- HRP: horseradish peroxidase
- HRV: human rhinovirus
- HRV-16: human rhinovirus 16
- ICAM-1: Intercellular adhesion molecule 1
- (IP)-10: interferon-gamma-inducible protein
- IRF-3/7: interferon regulatory factor 3/7
- JNK: c-Jun N-terminal kinase
- MAPK: mitogen activated protein kinase
- MAPKAP-2K: mitogen activated protein kinase activated protein 2 kinase
- Me: methyl
- MEK: map-Erk kinase
- MeOH: methanol
- min: minute(s)
- MOI: multiplicity of infection
- MTT: 3-(4,5-dimethylthiazol-2-yl)-2,5-diphenyltetrazolium bromide
- NFκB: nuclear factor kappa B
- Ph: phenyl
- PBS: phosphate buffered saline
- p38MAPKα: P38 Mitogen-activated protein kinases
- pMDI: pressurised meter dose inhaler
- RT: room temperature
- RT-PCR: real time-polymerase chain reasction
- SDS: sodium dodecyl sulfate
- SRC-1: cellular SRC
- SYK: spleen tyrosine kinase
- TCID₅₀: 50% tissue culture infectious doses
- TMB: 3,3',5,5'-tetramethylbenzidine
- TNFα: tumor necrosis factor alpha
- URTI(s): upper respiratory tract infection(s)

### EXAMPLES

### General Procedures

Generic synthetic methods suitable for the preparation of compounds of formula (I) and procedures relating to the synthesis of compound examples of the invention have been previously disclosed [Ito K. et al., WO 2010/067130, PCT/GB2009/051702, 17 June 2010] and are listed below (**Table 1**).

**Table 1**

| | | |
|---|---|---|
| **1** | 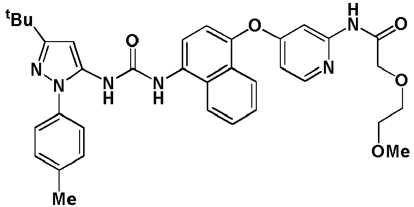 | *N*-(4-(4-(3-(3-*tert*-Butyl-1*-p-*tolyl-1*H*-pyrazol-5-yl)ureido) naphthalen-1-yloxy) pyridin-2-yl)-2-(2-methoxyethoxy) acetamide. |
| **2** | 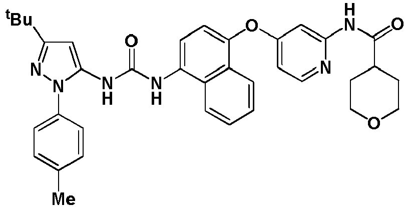 | *N*-(4-(4-(3-(3-*tert*-Butyl-1*-p-*tolyl-1*H*-pyrazol-5-yl)ureido) naphthalen-1-yloxy)pyridin-2-yl)tetrahydro-2*H*-pyran-4-carboxamide. |
| **3** | 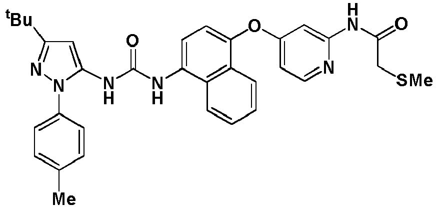 | *N*-(4-(4-(3-(3-*tert*-Butyl-1*-p-*tolyl-1*H*-pyrazol-5-yl)ureido) naphthalen-1-yloxy)pyridin-2-yl)-2-(methylthio)acetamide. |
| **4** | 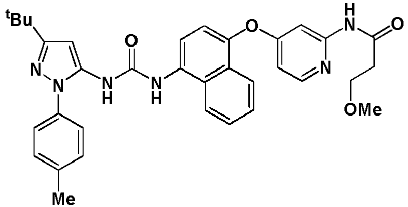 | *N*-(4-(4-(3-(3-*tert*-Butyl-1*-p-*tolyl-1*H*-pyrazol-5-yl)ureido) naphthalen-1-yloxy)pyridin-2-yl)-3-methoxypropanamide. |
| **5** | 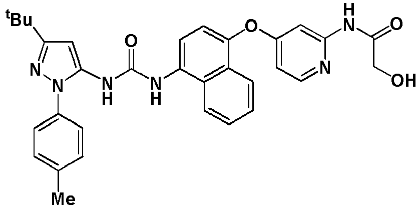 | *N*-(4-(4-(3-(3-*tert*-Butyl-1*-p-*tolyl-1*H*-pyrazol-5-yl)ureido) naphthalen-1-yloxy)pyridin-2-yl)-2-hydroxyacetamide. |
| **6** | 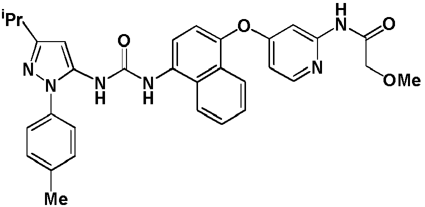 | *N*-(4-(4-(3-(3-Isopropyl-1-*p-*tolyl-1*H*-pyrazol-5-yl)ureido) naphthalen-1-yloxy)pyridin-2-yl)-2-methoxyacetamide. |
| **7** | 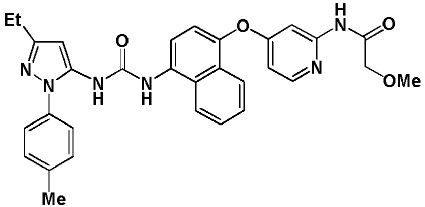 | *N*-(4-(4-(3-(3-Ethyl-1-*p*-tolyl-1*H-*pyrazol-5-yl)ureido) naphthalen-1-yloxy)pyridin-2-yl)-2-methoxyacetamide. |
| **8** | 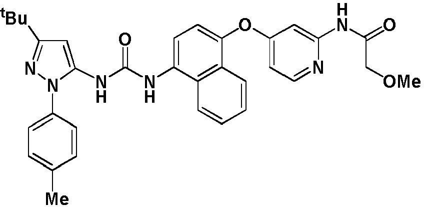 | *N*-(4-(4-(3-(3-*tert*-Butyl-1*-p-*tolyl-1*H*-pyrazol-5-yl)ureido) naphthalen-1-yloxy)pyridin-2-yl)-2-methoxyacetamide. |
| | | This compound is also referred to in the Biological Testing section as "Reference Compound 2" |
| **9** | 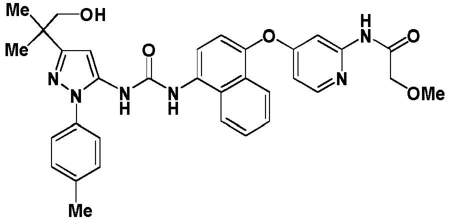 | *N*-(4-(4-(3-(3-(1-Hydroxy-2-methylpropan-2-yl)-1-*p*-tolyl-1*H*-pyrazol-5-yl)ureido) naphthalen-1-yloxy)pyridin-2-yl)-2-methoxyacetamide. |
| **10** | 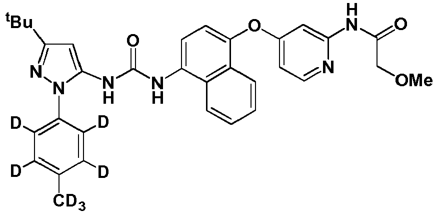 | *N*-(4-(4-(3-(3-*tert*-butyl-1-(2,3,5,6-tetradeutero-4-(trideuteromethyl)phenyl) -1*H*-pyrazol-5-yl)ureido) naphthalen-1-yloxy)pyridin-2-yl)-2-methoxyacetamide. |
| **11** | 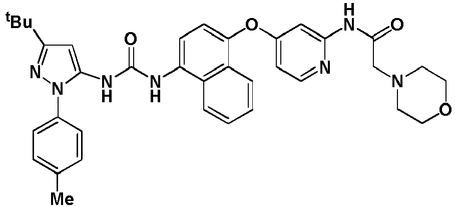 | *N*-(4-(4-(3-(3-*tert*-Butyl-1*-p-*tolyl-1 H-pyrazol-5-yl)ureido) naphthalen-1-yloxy)pyridin-2-yl)-2-morpholinoacetamide |
| **12** | 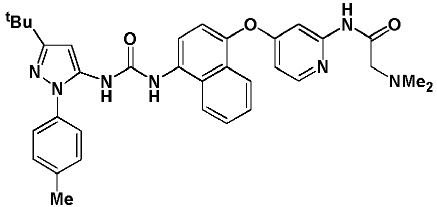 | *N*-(4-(4-(3-(3-*tert*-Butyl-1*-p-*tolyl-1*H*-pyrazol-5-yl)ureido) naphthalen-1-yloxy)pyridin-2-yl)-(dimethylamino)acetamide |
| **13** | 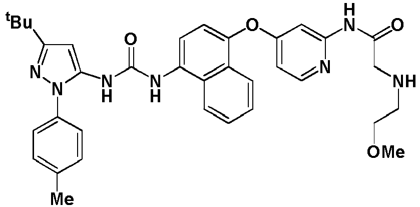 | *N*-(4-(4-(3-(3-*tert*-Butyl-1*-p-*tolyl-1*H*-pyrazol-5-yl)ureido) naphthalen-1-yloxy)pyridin-2-yl)-2-(2-methoxyethylamino) acetamide. |
| **14** | 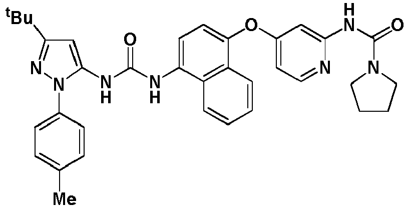 | *N*-(4-(4-(3-(3-*tert*-Butyl-1*-p-*tolyl-1*H*-pyrazol-5-yl)ureido) naphthalen-1-yloxy)pyridin-2-yl)pyrrolidine-1-carboxamide. |
| **15** | 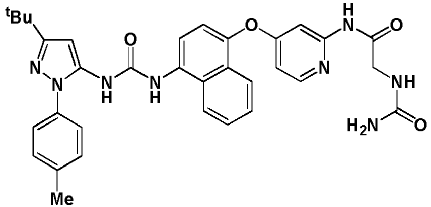 | *N*-(4-(4-(3-(3-*tert*-Butyl-1*-p-*tolyl-1*H*-pyrazol-5-yl)ureido) naphthalen-1-yloxy)pyridin-2-yl)-2-ureidoacetamide. |
| **16** | 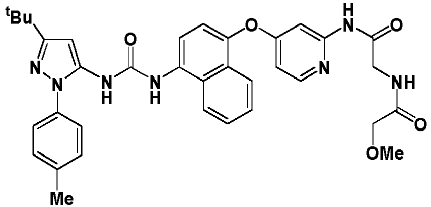 | *N*-(4-(4-(3-(3-*tert*-Butyl-1*-p-*tolyl-1*H*-pyrazol-5-yl)ureido) naphthalen-1-yloxy)pyridin-2-yl)-2-(2-methoxyacetamido) acetamide. |
| **17** | 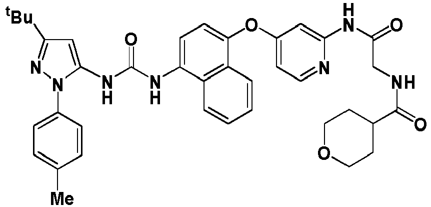 | *N*-(2-(4-(4-(3-(3-*tert*-Butyl-1*-p-*tolyl-1*H*-pyrazol-5-yl)ureido) naphthalen-1-yloxy)pyridin-2-ylamino)-2-oxoethyl)tetrahydro-2*H*-pyran-4-carboxamide. |
| **18** | 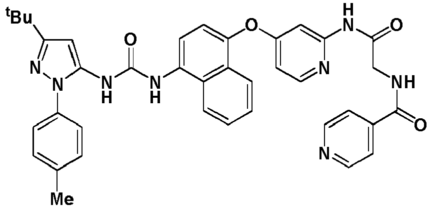 | *N*-(2-(4-(4-(3-(3-*tert*-Butyl-1*-p-*tolyl-1*H*-pyrazol-5-yl)ureido) naphthalen-1-yloxy)pyridin-2-ylamino)-2-oxoethyl) isonicotinamide |
| **19** | 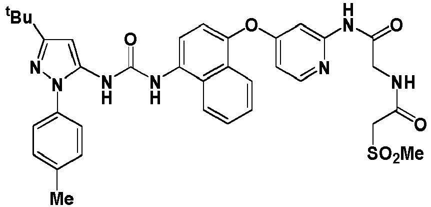 | *N*-(4-(4-(3-(3-*tert*-Butyl-1*-p-*tolyl-1*H*-pyrazol-5-yl)ureido) naphthalen-1-yloxy)pyridin-2-yl)-2-(2-(methylsulfonyl) acetamido)acetamide. |
| **20** | 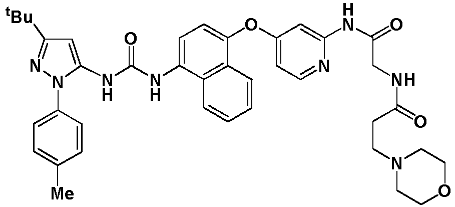 | *N*-(2-(4-(4-(3-(3-*tert*-Butyl-1*-p-*tolyl-1*H*-pyrazol-5-yl)ureido) naphthalen-1-yloxy)pyridin-2-ylamino)-2-oxoethyl)-3-morpholinopropanamide. |
| **21** | 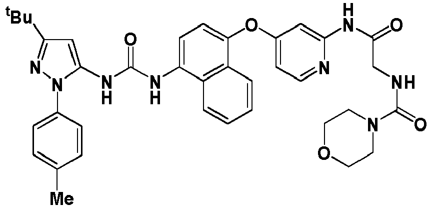 | *N*-(2-(4-(4-(3-(3-*tert*-Butyl-1*-p-*tolyl-1*H*-pyrazol-5-yl)ureido) naphthalen-1-yloxy)pyridin-2-ylamino)-2-oxoethyl)morpholine -4-carboxamide. |
| **22** | 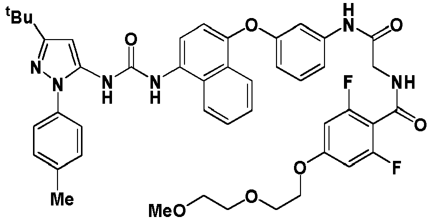 | *N*-(2-(4-(4-(3-(3-*tert*-Butyl-1*-p-*tolyl-1*H*-pyrazol-5-yl)ureido) naphthalen-1-yloxy)pyridin-2-ylamino)-2-oxoethyl)-2,6-difluoro-3-(2-(2-methoxyethoxy) ethoxy)benzamide. |
| **23** | 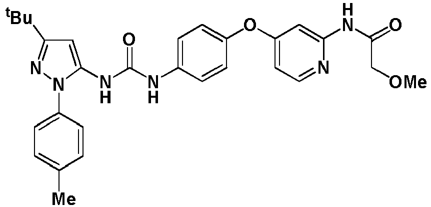 | *N*-(4-(4-(3-(3-*tert*-Butyl-1*-p-*tolyl-1*H*-pyrazol-5-yl)ureido) phenoxy)pyridin-2-yl)-2-methoxyacetamide. |
| **24** | 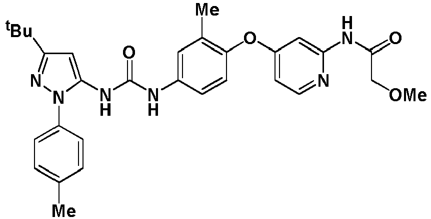 | *N*-(4-(4-(3-(3-*tert*-Butyl-1*-p-*tolyl-1*H*-pyrazol-5-yl)ureido)-2-methylphenoxy)pyridin-2-yl)-2-methoxyacetamide. |
| **25** | 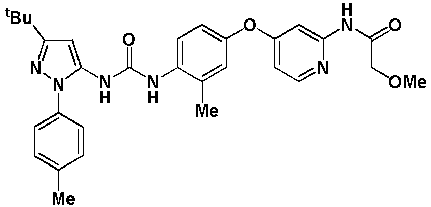 | *N*-(4-(4-(3-(3-*tert*-Butyl-1-*p-*tolyl-1*H*-pyrazol-5-yl)ureido)-3-methylphenoxy)pyridin-2-yl)-2-methoxyacetamide. |
| **26** | 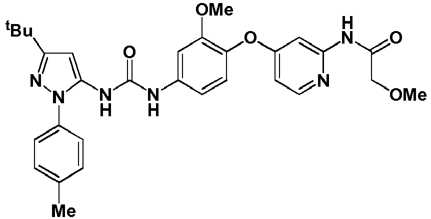 | *N*-(4-(4-(3-(3-*tert*-Butyl-1-*p-*tolyl-1*H*-pyrazol-5-yl)ureido)-2-methoxyphenoxy)pyridin-2-yl)-2-methoxyacetamide. |
| **27** | 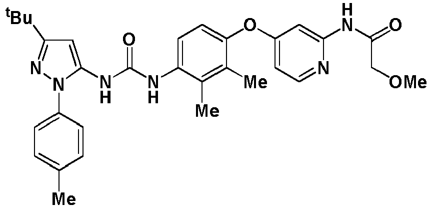 | *N*-(4-(4-(3-(3-*tert*-Butyl-1*-p-*tolyl-1*H*-pyrazol-5-yl)ureido)-2,3-dimethylphenoxy)pyridin-2-yl)-2-methoxyacetamide. |
| **28** | 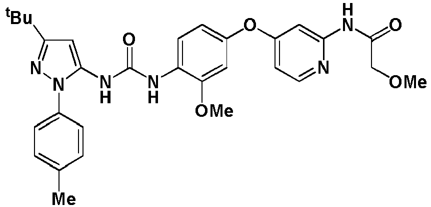 | *N*-(4-(4-(3-(3-*tert*-Butyl-1-*p-*tolyl-1*H*-pyrazol-5-yl)ureido)-3-methoxyphenoxy)pyridin-2-yl)-2-methoxyacetamide. |
| **29** | 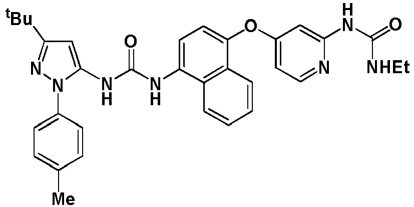 | *N*-Ethyl-*N*'-4-(4-(3-(3-*tert*-butyl-1-p-tolyl-1*H*-pyrazol-5-yl) ureido)naphthalen-1-yloxy) pyridin-2-ylurea. |
| **30** | 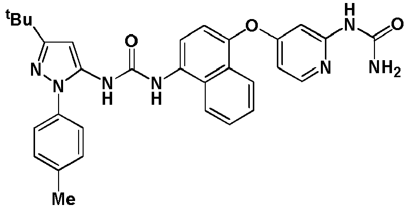 | 4-(4-(3-(3-*tert*-Butyl-1-*p*-tolyl-1*H*-pyrazol-5-yl)ureido) naphthalen-1-yloxy)pyridin-2-ylurea. |
| **31** | 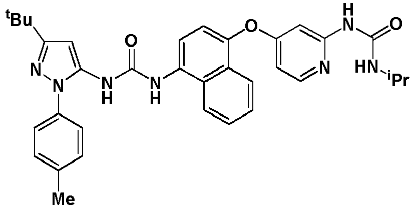 | *N*-Propan-2-yl-*N*'-4-(4-(3-(3-*tert*-butyl-1-*p*-tolyl-1*H*-pyrazol-5-yl)ureido)naphthalen-1-yloxy) pyridin-2-ylurea. |
| **32** | 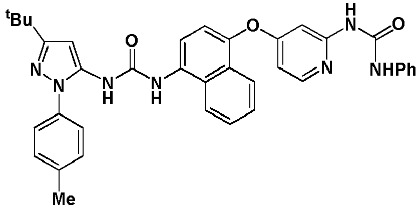 | 1-(3-(*tert*-Butyl)-1-(*p*-tolyl-1*H-*pyrazol-5-yl)-3-(4-((2-(3-phenyl ureido)pyridin-4-yl)oxy) naphthalen-1-yl)urea. |
| **33** | 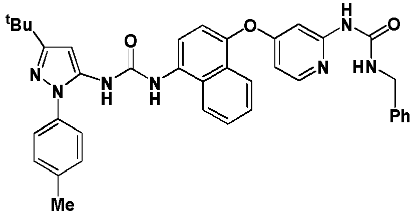 | 1-(4-((2-(3-Benzylureido) pyridin-4-yl)oxy)naphthalen-1-yl)-3-(3-(*tert*-butyl)-1-(*p*-tolyl)-1*H*-pyrazol-5-yl)urea. |
| **34** | 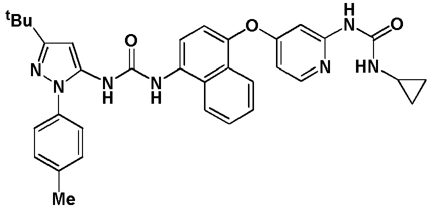 | 1-(4-((2-(3-Cyclopropylureido) pyridin-4-yl)oxy)naphthalen-1-yl)-3-(3-(*tert*-butyl)-1-(*p*-tolyl)-1*H*-pyrazol-5-yl)urea. |
| **35** | 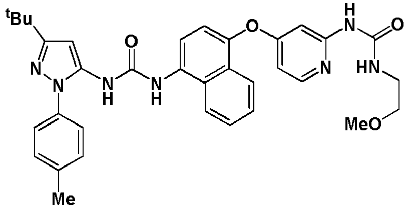 | 1-(3-(*tert*-Butyl)-1-(*p*-tolyl)-1*H-*pyrazol-5-yl)-3-(4-((2-(3-(2-methoxyethyl)ureido)pyridin-4-yl)oxy)naphthalen-1-yl)urea. |
| **36** | 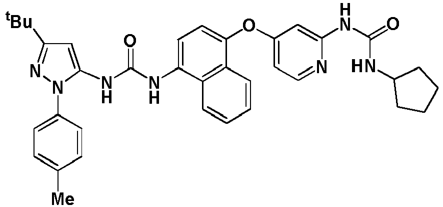 | 1-(3-(*tert*-Butyl)-1-(*p*-tolyl)-1*H-*pyrazol-5-yl)-3-(4-((2-(3-cyclopentyl)ureido) pyridin-4-yl)oxy)naphthalen-1-yl)urea. |
| **37** | 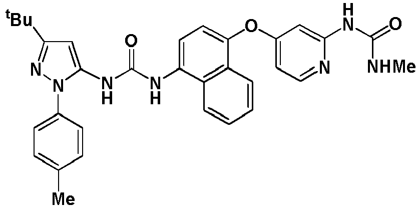 | 1-(3-(*tert*-Butyl)-1-(*p*-tolyl)-1*H-*pyrazol-5-yl)-3-(4-((2-(3-methyl) ureido)pyridin-4-yl)oxy) naphthalen-1-yl)urea. |
| **38** | 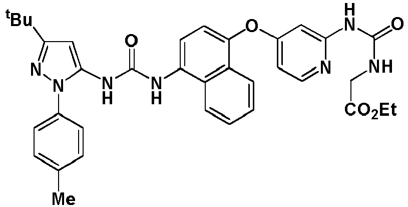 | Ethyl 2-(3-(4-((4-(3-(3-(*tert-*butyl)-1-(*p*-tolyl)-1*H*-pyrazol-5-yl)ureido)naphthalen-1-yl)oxy) pyridin-2-yl)ureido)acetate. |
| **39** | 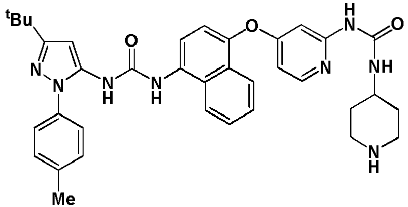 | 4-(3-(4-(4-(3-(3-*tert*-Butyl-1-*p-*tolyl-1*H*-pyrazol-5-yl)ureido) naphthalen-1-yloxy)pyridin-2-yl)ureido)piperidine. |
| **40** | 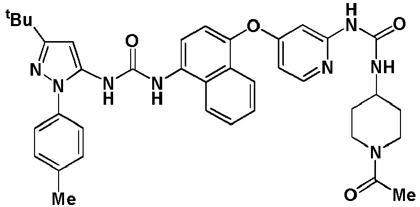 H ᵗBu O O N O N NH NH N NH N H H N Me O Me | *N*-Acetyl4-(3-(4-(4-(3-(3-*tert-*butyl-1-*p*-tolyl-1*H*-pyrazol-5-yl)ureido)naphthalen-1-yloxy) pyridin-2-yl)ureido)piperidine. |
| **41** | 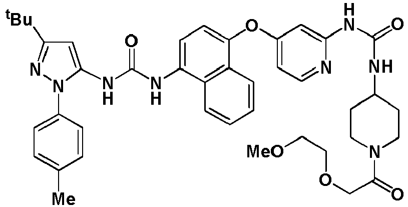 | 2-(2-Methoxyethoxy)-1-(4-(3-(4-(4-(3-(3-*tert*-butyl-1-*p*-tolyl-1*H-*pyrazol-5-yl)ureido)naphthalen-1-yloxy)pyridin-2-yl)ureido) piperidin-1-yl)ethanone. |
| **42** | 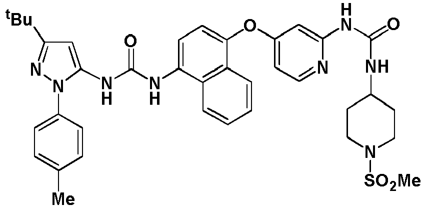 tBu O N N N H N H O H N O N N H N SO2Me Me | *N*-Methylsulfonyl-4-(3-(4-(4-(3-(3-*tert*-butyl-1-*p*-tolyl-1*H-*pyrazol-5-yl)ureido)naphthalen-1-yloxy)pyridin-2-yl)ureido) piperidine. |
| **43** | 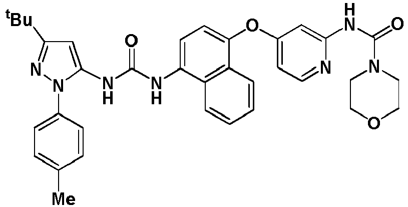 | *N*-(4-(4-(3-(3-*tert*-Butyl-1*-p-*tolyl-1*H*-pyrazol-5-yl)ureido) naphthalen-1-yloxy)pyridin-2-yl)morpholine-4-carboxamide. |
| **44** | 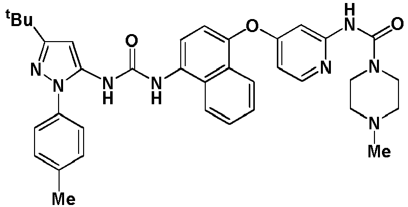 | *N*-(4-((4-(3-(3-(*tert*-Butyl)-1-(*p-*tolyl)-1*H*-pyrazol-5-yl)ureido) naphthalen-1-yl)oxy)pyridin-2-yl)-4-methylpiperazine-1-carboxamide. |
| **45** | 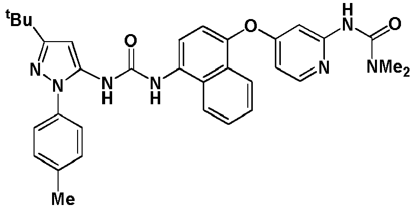 | 3-(4-((4-(3-(3-(*tert*-Butyl)-1-(*p-*tolyl)-1*H*-pyrazol-5-yl)ureido) naphthalen-1-yl)oxy)pyridin-2-yl)-1,1-dimethylurea. |
| **46** | 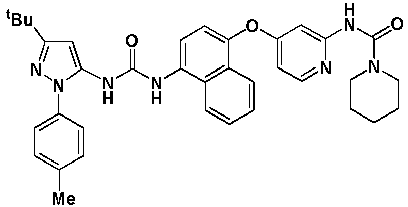 | *N*-(4-((4-(3-(3-(*tert*-Butyl)-1-(*p-*tolyl)-1*H*-pyrazol-5-yl)ureido) naphthalen-1-yl)oxy)pyridin-2-yl)piperidine-1-carboxamide. |
| **47** | 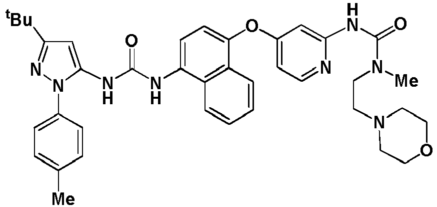 | *N*-Methyl-*N*-(2-(morpholin-4-yl)ethyl)-*N*'-4-(4-(3-(3-*tert*-butyl-1-p-tolyl-1*H*-pyrazol-5-yl) ureido)naphthalen-1-yloxy) pyridin-2-ylurea. |
| **48** | 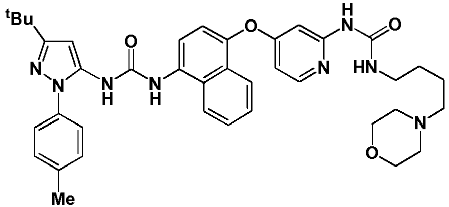 | *N*-(4-(Morpholin-4-yl)butyl)-*N*'-4-(4-(3-(3-*tert*-butyl-1-*p*-tolyl-1*H*-pyrazol-5-yl)ureido) naphthalen-1-yloxy)pyridin-2-ylurea. |
| **49** | 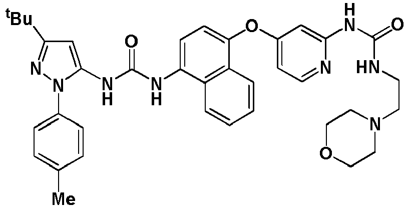 | *N*-(2-(Morpholin-4-yl)ethyl)-*N*'-4-(4-(3-(3-*tert*-butyl-1-*p*-tolyl-1*H*-pyrazol-5-yl)ureido) naphthalen-1-yloxy)pyridin-2-ylurea. |
| **50** | 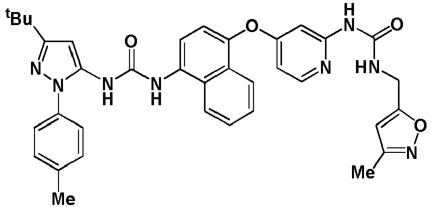 | *N*-(3-Methylisoxazol-5-yl) methyl-*N*'-4-(4-(3-(3-*tert*-butyl-1-*p*-tolyl-1*H*-pyrazol-5-yl) ureido)naphthalen-1-yloxy) pyridin-2-ylurea. |
| **51** | 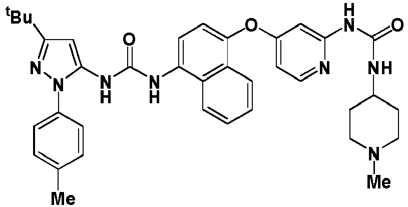 | *N*-(1-Methyl)piperidin-4-yl-*N'*-4-(4-(3-(3-*tert*-butyl-1-*p*-tolyl-1*H-*pyrazol-5-yl)ureido)naphthalen-1-yloxy)pyridin-2-ylurea. |
| **52** | 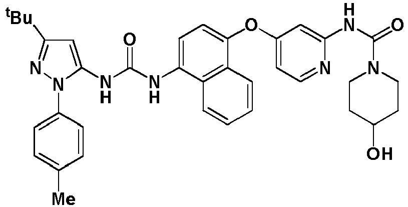 | *N*-(4-(4-(3-(3-*tert*-Butyl-1*-p-*tolyl-1*H*-pyrazol-5-yl)ureido) naphthalen-1-yloxy)pyridin-2-yl)-4-hydroxypiperidine-1-carboxamide. |
| **53** | 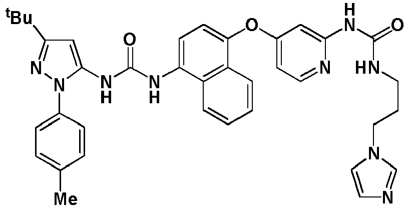 | *N*-(3-(Imidazol-1-yl)propyl)-*N*'-4-(4-(3-(3-*tert*-butyl-1-*p*-tolyl-1*H*-pyrazol-5-yl)ureido) naphthalen-1-yloxy)pyridin-2-ylurea. |
| **54** | 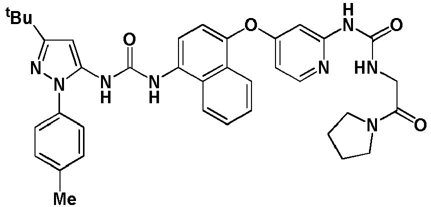 | *N*-(2-(3-(4-(4-(3-(3-*tert*-Butyl-1-*p*-tolyl-1*H*-pyrazol-5-yl)ureido) naphthalen-1-yloxy)pyridin-2-yl)ureido)acetyl)pyrrolidine. |
| **55** | 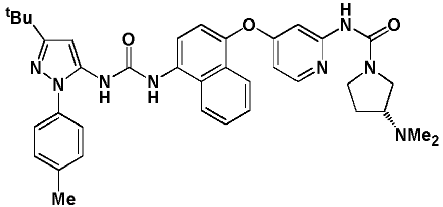 | (*R*)-*N*-(4-(4-(3-(3-*tert*-Butyl-1-*p-*tolyl-1*H*-pyrazol-5-yl)ureido) naphthalen-1-yloxy)pyridin-2-yl)-3-(dimethylamino) pyrrolidine-1-carboxamide. |
| **56** | 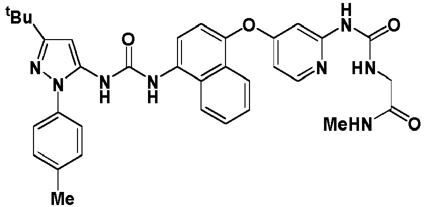 | 2-(3-(4-(4-(3-(3-*tert*-Butyl-1-*p-*tolyl-1*H*-pyrazol-5-yl)ureido) naphthalen-1-yloxy)pyridin-2-yl)ureido)-*N*-methylacetamide. |
| **57** | 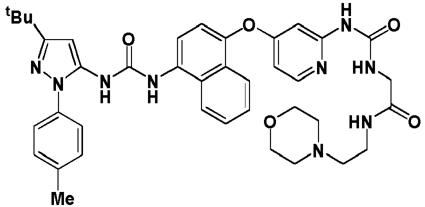 | 2-(3-(4-(4-(3-(3-*tert*-Butyl-1-*p-*tolyl-1*H*-pyrazol-5-yl)ureido) naphthalen-1-yloxy)pyridin-2-yl)ureido)-*N*-(2-morpholino ethyl)acetamide. |
| **58** | 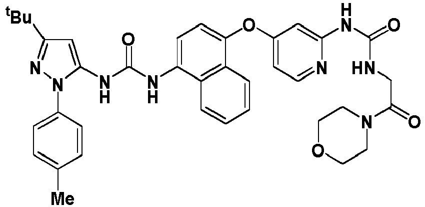 | 2-(3-(4-(4-(3-(3-*tert*-Butyl-1-*p-*tolyl-1*H*-pyrazol-5-yl)ureido) naphthalen-1-yloxy)pyridin-2-yl)ureido)acetyl morpholine. |
| **59** | 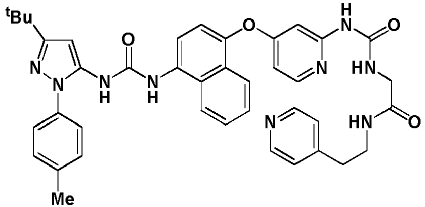 | 2-(3-(4-(4-(3-(3-*tert*-Butyl-1-*p-*tolyl-1*H*-pyrazol-5-yl)ureido) naphthalen-1-yloxy)pyridin-2-yl)ureido)-*N*-(2-(pyridin-4-yl) ethyl)acetamide. |
| **60** | 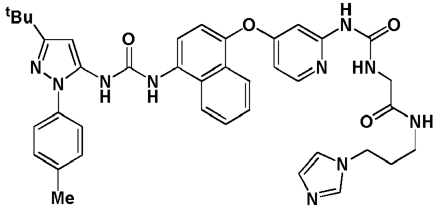 | *N*-(3-(1*H*-Imidazol-1-yl)propyl)-2-(3-(4-(4-(3-(3-*tert*-butyl-1*-p-*tolyl-1*H*-pyrazol-5-yl)ureido) naphthalen-1-yloxy)pyridin-2-yl) ureido)acetamide. |
| **61** | 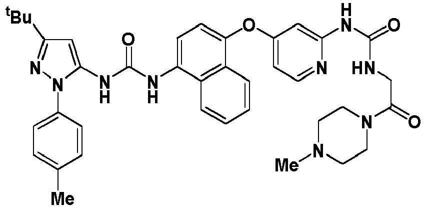 | 1-(2-(3-(4-(4-(3-(3-*tert*-Butyl-1-*p*-tolyl-1H-pyrazol-5-yl)ureido) naphthalen-1-yloxy)pyridin-2-yl)ureido)acetyl)-4-methyl piperazine. |
| **62** | 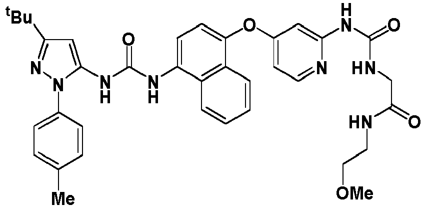 | *N*-(3-(1*H*-Imidazol-1-yl)propyl)-2-(3-(4-(4-(3-(3-*tert*-butyl-1*-p-*tolyl-1*H*-pyrazol-5-yl)ureido) naphthalen-1-yloxy)pyridin-2-yl) ureido)acetamide. |
| **63** | 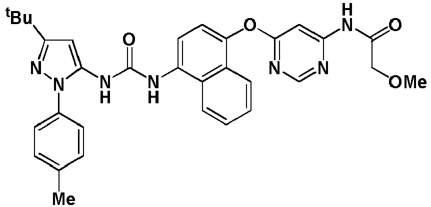 | N-(6-(4-(3-(3-*tert*-Butyl-1-p-tolyl-1*H*-pyrazol-5-yl)ureido) naphthalen-1-yloxy)pyrimidin-4-yl)-2-methoxyacetamide. |
| **64** | 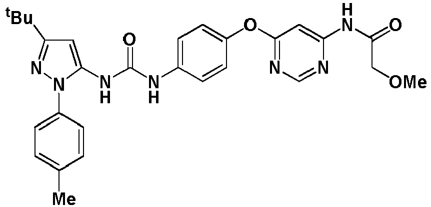 | N-(6-(4-(3-(3-*tert*-Butyl-1-p-tolyl-1*H*-pyrazol-5-yl)ureido) phenoxy)pyrimidin-4-yl)-2-methoxyacetamide. |
| **65** | 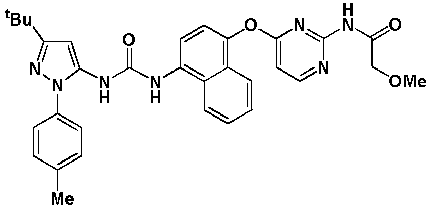 | *N*-(4-(4-(3-(3-*tert*-Butyl-1*-p-*tolyl-1*H*-pyrazol-5-yl)ureido) naphthalen-1-yloxy)pyrimidin-2-yl)-2-methoxyacetamide. |
| **66** | 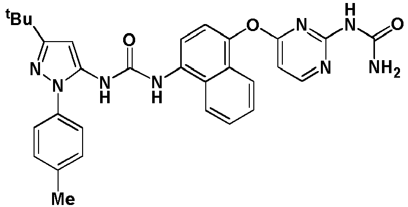 | 3-(4-(4-(3-(3-*tert*-Butyl-1-*p*-tolyl-1*H*-pyrazol-5-yl)ureido) naphthalen-1-yloxy)pyrimidin-2-yl)urea. |
| **67** | 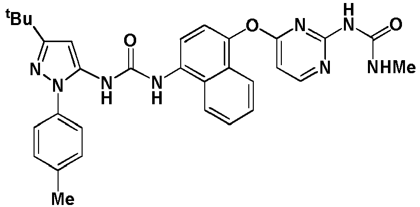 | 1-Methyl-3-(4-(4-(3-(3-*tert-*butyl-1-*p*-tolyl-1*H*-pyrazol-5-yl)ureido)naphthalen-1-yloxy) pyrimidin-2-yl)urea. |
| **68** | 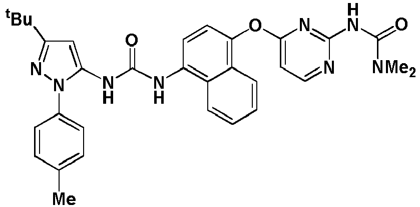 | 1,1-Dimethyl-3-(4-(4-(3-(3-*tert-*butyl-1-*p*-tolyl-1*H*-pyrazol-5-yl) ureido)naphthalen-1-yloxy) pyrimidin-2-yl)urea. |
| **69** | 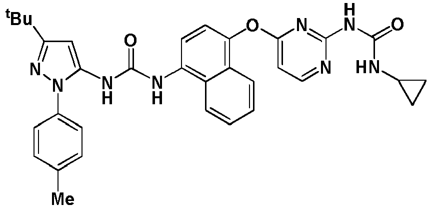 | Cyclopropyl-3-(4-(4-(3-(3-*tert-*butyl-1-*p*-tolyl-1*H*-pyrazol-5-yl) ureido)naphthalen-1-yloxy) pyrimidin-2-yl)urea. |
| **70** | 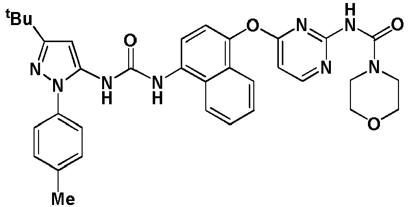 | (4-(4-(3-(3-*tert*-Butyl-1-*p*-tolyl-1*H*-pyrazol-5-yl)ureido) naphthalen-1-yloxy)pyrimidin-2-yl)morpholine-4-carboxamide. |
| **71** | 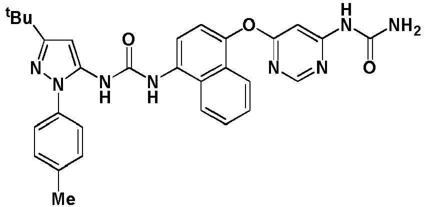 | 3-(6-(4-(3-(3-*tert*-Butyl-1-*p*-tolyl-1*H*-pyrazol-5-yl)ureido) naphthalen-1-yloxy)pyrimidin-4-yl)urea. |

### Biological Testing

Pleconaril is an anti-viral drug targeting picornaviruses, including rhinovirus, and is effective by preventing viral entry into cells. Clinical development of the compound has demonstrated that it is active against rhinovirus infections (Jefferson T.O. and Tyrrell D., 2007; Cochrane Database Syst Rev 18, CD002743). The characteristics of pleconaril and various compounds were investigated further in the *in vitro* assays described herein.

Rhinovirus is able to propagate in a range of cells found in the airways, including fibroblasts and epithelial cells. The effects of a number of kinase inhibitors on virus propagation was investigated using viral load as a biomarker in a human fibroblast cell line, MRC-5, and in primary human epithelial cells (*ex*. Mattek Corporation, Epithelix Sarl.), as well as on the induction of ICAM-1 and release of IL-8 as markers of a pro-inflammatory response in a human bronchial epithelial cell line, BEAS2B. Using PCR detection, the effect of treatments on the production of interferon β in cells in the basal condition and following inoculation with virus, was also investigated.

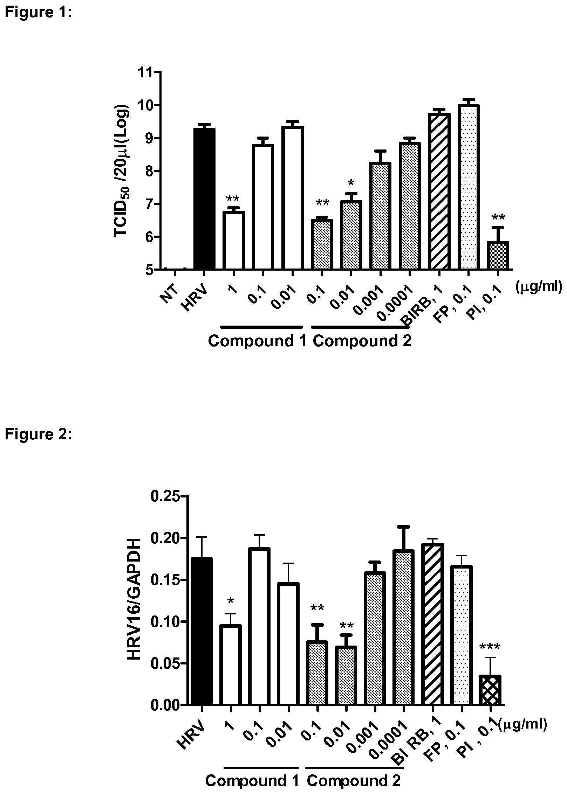

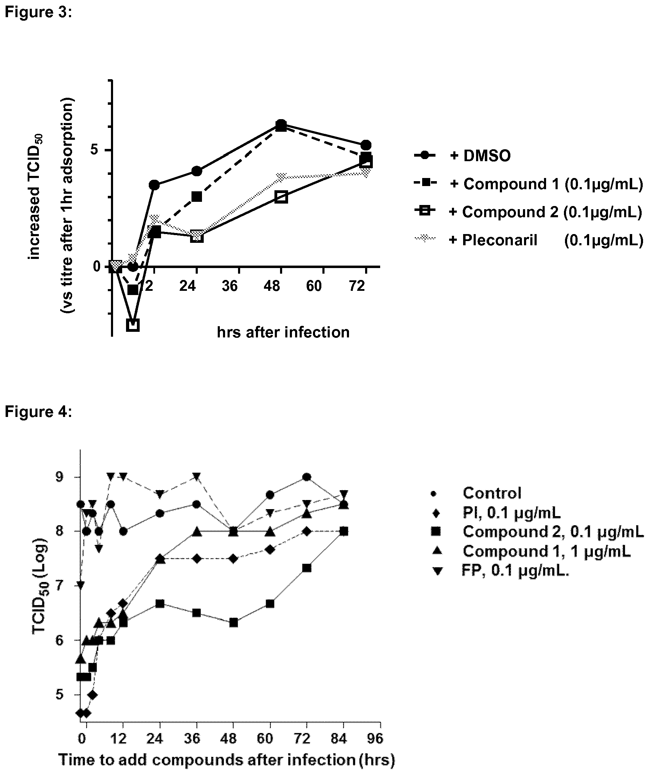

The same compounds have been evaluated for their ability to decrease rhinovirus load following infection of MRC5 cells with HRV16 for 3 days (0.1 MOI). The results demonstrate that pleconaril clearly inhibits both extracellular HRV loads and detectable HRV16 mRNA in cellular extracts. By contrast, fluticasone propionate was without effect. Reference Compound 1 (*N*-[4-({4-[3-(3-*tert*-Butyl-1-*p*-tolyl-1*H*-pyrazol-5-yl)ureido]naphthalen-1-yloxy}methyl)pyridin-2-yl]-2-methoxyacetamide, not within the scope of compounds of Formula (I)) showed a modest effect on HRV16 replication and Reference Compound 2 (*N-*[4-{4-[3-(3-*tert*-Butyl-1-*p*-tolyl-1*H*-pyrazol-5-yl)ureido]naphthalen-1-yloxy}pyridin-2-yl]-2-methoxyacetamide = **Example 8)** was found to be a potent inhibitor of the accumulation of extracellular HRV16 load (**Figure 1**) and of HRV16 mRNA detected in cellular extracts (**Figure 2**). Furthermore, Reference Compound 2 was found to be a potent inhibitor of HRV39 extracellular virus load in cultured nasal epithelial cells (air liquid interface), whereas Reference Compound 1 had little effect (**Figure 3**).

The effect of treatment time with Reference Compound 1 and Reference Compound 2 *versus* the point of inoculation with the virus HRV16, was then investigated. In pilot studies it was confirmed that pleconaril was essentially only effective if the drug was administered prior to infection with HRV16, as was expected from its mechanism of action (**Figure 4**).

The impact of treatment timing on the effects of Reference Compound 1 and Reference Compound 2 on HRV16 viral replication was investigated and the results shown herein below (**Figure 4**). The data demonstrate that in MRC5 cells infected with HRV16 (0.1 MOI) and followed for a total period of 84 hr, both Reference Compound 1 and Reference Compound 2 initially showed marked activity *versus* extracellular HRV16 load. Delaying treatment for 24-36 hr with Reference Compound 1 resulted in a marked decrease in activity, whereas activity with Reference Compound 2 was more sustained.

The profile shown by Reference Compound 2 in this study provides evidence that compared to Reference Compound 1, it is superior as a therapeutic agent for treating HRV infection.

The effects of compounds related to Reference Compound 2 on HRV-induced cytopathic effects (CPE) in MRC5 cells were also investigated. Examples 30, 43, 44, 45, 37, 55 and 14 inhibited HRV16-induced CPE in MRC5 cells in a concentration-dependent manner and showed similar efficacy to Reference Compound 2 (see **Table 1**).

**Table 1**

| **Test Material Compound Example** | **HRV16-induced CPE IC₅₀ Value (nM)** | **HRV16-induced IL-8 IC₅₀ Value (nM)** | **HRV16-induced ICAM1 IC₅₀ Value (nM)** |
|---|---|---|---|
| **Ref. Compound 1^{a}** | 340 | 1.0 | >1689 |
| **Ref. Compound 2^{b}** | 4.7 | 0.065 | 0.37 |
| **30** | 2.9 | 0.019 | ND |
| **43** | 9.5 | 0.069 | ND |
| **44** | 16.3 | 0.041 | 0.069 |
| **45** | 9.4 | 0.041 | 0.098 |
| **37** | 24.0 | 0.12 | 0.029 |
| **55** | 14.3 | ND | ND |
| **14** | 3.3 | ND | ND |

| | | | |
|---|---|---|---|
| **a**) *N*-[4-({4-[3-(3-*tert*-Butyl-1-*p*-tolyl-1*H*-pyrazol-5-yl)ureido]naphthalen-1-yloxy}methyl)pyridin-2-yl]-2-methoxyacetamide; **b**) *N*-[4-{4-[3-(3-*tert*-Butyl-1-*p*-tolyl-1*H*-pyrazol-5-yl)ureido]naphthalen-1-yloxy}pyridin-2-yl]-2-methoxyacetamide = **Example 8; ND:** not determined. | | | |

Since binding to ICAM-1 is a mechanism of cellular entry which is exploited by major group rhinovirus strains, we examined the effects of treatment on this response. Reference Compound 1 was found to have no effect on ICAM-1 expression induced by HRV16 inoculation (2 hr infection) in BEAS2B cells. However, Reference Compound 2 showed a remarkably potent inhibitory effect (IC₅₀ = 0.37 nM). By comparison, the effects of fluticasone propionate were much more modest, concentration-dependent effects only being apparent at 10,000-fold higher concentrations than for Reference Compound 2 (results not shown). Treatment of epithelial cells with either Reference Compound 1 or Reference Compound 2 was found to produce marked inhibition of IL-8 production in response to inoculation using either HRV-16 (**Table 1**) or HRV39 (**Figure 5**).

RNA virus signalling within infected cells is complex, but may involve activation of both NFκB and IRF-3. Single-stranded RNA from virus, or double-stranded RNA processed after viral entry, stimulate viral RNA receptors such as RIG-I, MDA5, or TLR3. These stimulated responses cause activation of either IRF3/7 or NFκB. Activation of NFκB promotes the release of pro-inflammatory cytokines, with some stimulatory effects on caspase and interferon production. Interestingly, activation of IRF3/7 can lead to production of anti-viral proteins directly or independently of interferon production and signalling (**Figure 6**).

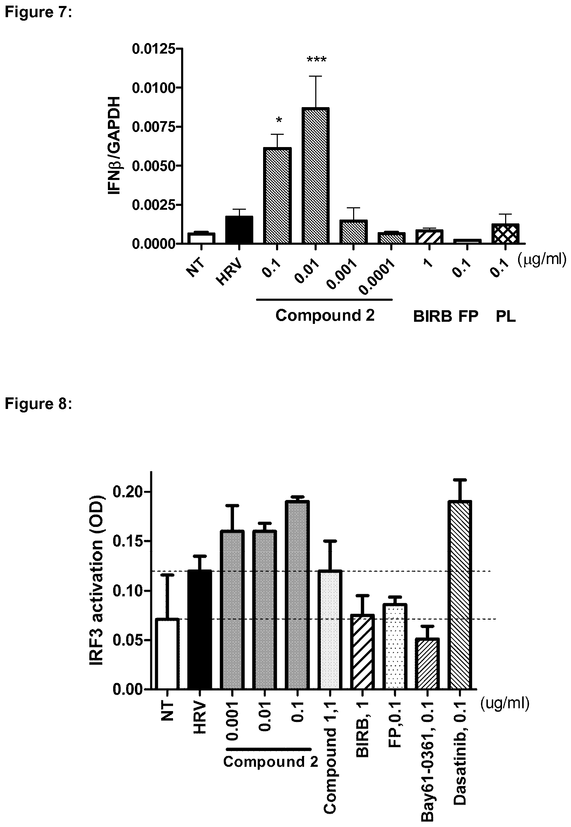

The effects of treatment with Reference Compound 1, Reference Compound 2, pleconaril, the pan p38 inhibitor **BIRB 796,** (a standard p38MAPK inhibitor) or fluticasone propionate on HRV-16-induced interferon β induction by HRV16 was examined. The results show that of the treatments evaluated, Reference Compound 2 was distinguished by its ability to produce marked increases in interferon β induction (**Figure 7**). In subsequent experiments, it was demonstrated that HRV-induced interferon production limited HRV16 virus load in MRC-5, cells since the inclusion of an interferon β- neutralising antibody in the incubation medium was found to increase virus load by approximately one to two log orders (results not shown). This data strongly suggests that induction of interferon β by Reference Compound 2 in infected MRC-5 cells is likely to play a role in the compound's ability to decrease HRV16 virus load.

The literature provides a number of indications that activation of host tyrosine kinase enzymes may play important roles in the processes which eventually lead to release of progeny virus from cells infected by HRV. However, there is no clear definition of the role played by particular kinase enzymes. Accordingly, compounds of the formula (**I**) were initially evaluated for their ability to inhibit a range of tyrosine kinase enzymes in cell free systems and the results are presented below (**Table 2**).

**Table 2: Inhibitory activity of selected test compounds versus a panel of kinase enzymes.**

| **Test Compounds** | **Enzyme assay: IC₅₀ values (nM)** | | | |
|---|---|---|---|---|
| | **p38MAPKα** | **HCK** | **c-SRC** | **SYK** |
| **Reference Compound 1** | 5 | 47 | 162 | >17794 |
| **Reference Compound 2** | 12 | 3 | 5 | 42 |
| **BIRB-796** (pan p38MAPK inhibitor) | 12 | >1894 | >1894 | >18940 |
| **BAY 61-3606** (SYK inhibitor) | >3313 | >3313 | >3313 | 59 |
| **Dasatinib** (SRC family kinase inhibitor) | 11 | 2 | 0.4 | >2436 |

The results obtained for Reference Compound 1 and Reference Compound 2 are broadly in line with Kd estimates evaluated by comprehensive kinase profiling (KINOME Scan, Ambit Biosciences, SanDiego, CA (results not shown).

HRV-16 has been reported to induce activation of NFκB, and be able to induced cytokine release through both NFκB-independent (IL-6, IL-8) and (at least partially) dependent mechanisms (GM-CSF) from human airway epithelial cells (Kim J., Sanders, S.P. et al., J. Immunol., 2000, 165:3384-3392.). In breast epithelial cells, SYK enhances TNFα-induced activation of NFκB (Zhou Q. and Geahlen R.L., Oncogene, 2009, 28:1348-1356.). In Jurkat T-cells TNFα-induced activation of NFκB was also reported to be dependent upon signalling through SYK (Takada Y. and Aggarwal B.B., J. Immunol., 2004, 173:1066-1077.). HRV has been reported to disrupt IRF-3 activation in HeLa cells (Peng T., Kotla S. et al., J. Virol., 2006, 80:5021-5031.). Accordingly the effects of the compounds listed in Table 2 on HRV-16 induced NFκB and IRF-3 activation were investigated.

The effects of treatment on HRV-16-induced activation of IRF-3 in BEAS2B cells (after 2 hr, at 10 MOI), was investigated. The results demonstrate that, of the treatments, only Reference Compound 2 and dasatinib enhanced HRV-16 induced activation of IRF-3 (**Figure 8**). In contrast, Reference Compound 1 was without effect, whereas **BIRB-796,** fluticasone propionate and BAY 61-036 showed a tendency to inhibit IRF-3 activation.

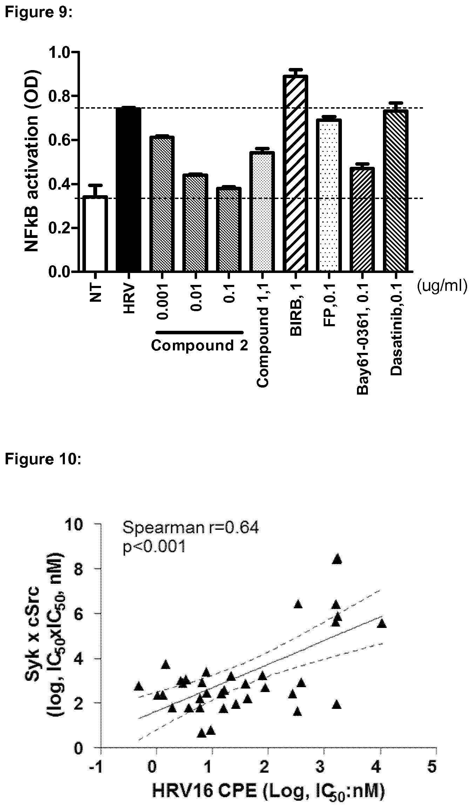

The effect of each of the treatments on HRV-16 induced activation of NFκB in BEAS2B cells (after 2 hr, at 10 MOI;) was examined. It was established that Reference Compound 2 and SYK inhibitors such as BAY 61-036 and R-406 inhibited HRV-16 induced NFκB-induced activation. Reference Compound 1 produced some evidence of inhibition of HRV-16 induced NFκB-induced activation whereas **BIRB-796,** fluticasone propionate and dasatinib had no clear effect on this response (**Figures 9**). These results are summarised below (**Table 3**).

**Table 3: Effects of treatments on virus load and activation of NFκB or IRF-3.**

| **Treatment** | **HRV-16 virus load** | **NFκB activation** | **IRF-3 activation** |
|---|---|---|---|
| **Fluticasone propionate** | No effect | No effect | Modest inhibition |
| **BIRB-796** | No effect | No effect | Modest inhibition |
| **BAY 61-3606 / R-406** | Modest inhibition | Modest inhibition | Modest inhibition |
| **Dasatinib** | Modest inhibition | No effect | Activation |
| **BAY 61-3606 / R-406 + Dasatinib** | Marked inhibition | Marked inhibition | Activation |
| **Reference Compound 1** | Modest inhibition | Modest inhibition | No effect |
| **Reference Compound 2** | Marked inhibition | Marked inhibition | Activation |

### c-SRC /SYK plot versus potency on HRV-16 virus load.

As the combination of dasatinib (a SRC family kinase inhibitor) and Bay61-0361/R-406 (a SYK inhibitor) showed better efficacy against HRV16 viral load, the relationship between the product of c-SRC and SYK inhibitory activities and that of anti-HRV activity (CPE in MRC5) was analysed. This revealed that there was a good correlation between the index and CPE inhibition, suggesting that simultaneous inhibition of both c-SRC and SYK results in potent anti-HRV activity, mimicking the profile of Reference Compound 2 (**Figure 10**).

In order to determine whether these results have broad applicability to other rhinovirus strains additional experiments were conducted using HRV 1B and HRV39. These studies confirmed that Reference Compound 2 is a potent inhibitor of HRV1B and HRV39 titres whereas Reference Compound 1 had little or no discernable effect (MRC-5 cells, 0.1 or 10 MOI, 3 days; results not shown).

The results presented herein demonstrate that effective inhibition of HRV-16 virus load *in vitro* following infection of cells depends upon the simultaneous inhibition of both c-SRC and SYK, enzymes, irrespective of whether the pharmacology is delivered through the actions of a single, dual inhibitory compound or by two compounds, acting selectively on the individual kinases.

Thus, in one embodiment the present invention provides a compound or composition comprising of an inhibitor of SRC and SYK, in particular for the treatment or prevention of HRV infection.

### HRV-16 infection, assessment of virus load.

Human rhinovirus RV16 (HRV16) was obtained from the American Type Culture Collection (Manassas, VA). Viral stocks were generated by infecting Hela cells with HRV until 80% of the cells were cytopathic. MRC5 cells (human lung fibroblast, ATCC) were infected at 0.1 MOI (multiplicity of infection of 0.1) of HRV16 and incubated for 1 hr at 33°C with gentle shaking to stimulate adsorption. The cells were then washed with PBS, fresh media (DMEM-5% FCS) was added and the cells were incubated for a further 72 hr. Supernatant (20 µL) was collected and 10-fold serial dilutions were prepared with DMEM containing 1% FCS. All titrations were performed by infecting confluent Hela cell monolayers with serially diluted supernatant (10⁻¹-10⁻⁵) and then the cytopathic effect at each dilution 3 days after infection assessed by visual inspection. The cells were then stained with Crystal Violet for confirmation. The amount of virus required to infect 50% of Hela cells was calculated in each treatment as TCID₅₀ (50% tissue culture infectious dose, U(unit)/20 µL). Compounds were added 2 hr before HRV16 infection and 1 hr after infection when non-infected HRV was washed out.

### Assessment of HRV16 induced CPE in MRC5.

MRC-5 cells were infected with HRV16 (MOI of 1) in DMEM containing 5% FCS and 1.5 mM MgCl₂, followed by incubation for 1 hr at 33°C to stimulate adsorption. The supernatants were aspirated and fresh media was added and the preparations were then incubated for 4 days. Where appropriate, cells were pre-incubated with the test compound or DMSO for 2 hr, and the compounds and DMSO added again after washout of the virus. Supernatants were aspirated and incubated with methylene blue solution (2% formaldehyde, 10% methanol and 0.175% Methylene Blue) for 2 hr at RT. After washing, 1% SDS was added to each well, and plates were shaken lightly for 1-2 hr prior to reading the absorbance at 660 nm. The percentage inhibition for each well was calculated. The IC₅₀ value was calculated from the concentration-response curve generated from the serial dilutions of the test compound.

### Assessment of transcripts of HRV16 and interferon β by PCR.

The MRC5 cells infected with virus (as described above) were used for viral RNA or mRNA detection. Total RNA extraction and reverse transcription were performed using TaqMan®Fast Cells-to-CT™ Kit (Ambion Inc. Austin TX). The gene transcript level of HRV16 (PrimerDesign Ltd, Southampton, UK), IFNβ (Applied Biosystems, Warrington, UK) and GAPDH, as a house-keeping gene (PrimerDesign Ltd, Southampton, UK) were quantified by real-time PCR using commercially available primers on a StepOnePlus™ RT-PCR system (Applied Biosystems, Warrington, UK). Variations in cDNA concentrations between different samples were corrected using the housekeeping gene, whereby the GAPDH concentration in each cDNA sample was calculated, and the cDNA diluted to contain equal amounts of GAPDH. Standard curves for GAPDH were generated by performing a dilution series of the untreated control cDNA. The relative amount of gene transcript present after different treatments was calculated and normalized by dividing the calculated value for the gene of interest by that of the house-keeping gene value.

### Assessment of IL-8 release and ICAM1 expression activated by human rhinovirus.

BEAS2B cells were infected with rhinovirus at an MOI of 5 and incubated for 2 hr at 33°C with gentle shaking to enhance adsorption. Test compounds were added 2 hr before HRV infection and 2 hr after infection when residual extracellular HRV was washed out. The cells were then washed with PBS, fresh media was added and the cells were incubated for a further 72 hr. The supernatant was collected for assay of IL-8 concentrations using the Duoset ELISA development kit (R&D Systems, Minneapolis, MN).

The level of ICAM-1 expression on the cell surface was determined by cell-based ELISA. After appropriate incubation, cells were fixed with 4% formaldehyde in PBS. After quenching endogenous peroxidase by adding 0.1% sodium azide and 1% hydrogen peroxide, wells were washed with buffer (0.05% Tween in PBS: PBS-Tween). After incubation with the blocking solution (5% milk in PBS-Tween for 1 hr), cells were incubated with anti-human ICAM-1 antibody in 5% BSA PBS-Tween (1:500) overnight. Wells were then washed with PBS-Tween and incubated with the secondary antibody (HRP-conjugated anti-rabbit IgG, Dako Ltd.). The ICAM-1 signal was detected by adding substrate and the optical densities were read at a wavelength of 450 nm and at a reference wavelength of 655 nm using a spectrophotometer. The wells were then washed with PBS-Tween and total cell numbers in each well were determined by reading the absorbance at 595 nm after Crystal Violet staining and elution by 1% SDS solution. The measured OD 450-655 readings were corrected for cell number by dividing with the OD₅₉₅ reading in each well.

### HRV-39 viral load and IL-8 production using air-liquid interface cultured bronchial epithelial cells

Primary nasal epithelial cells, cultured using an air-liquid interface, were purchased from Epithelix Sari (Geneva, Switzerland). Fresh, warmed media (200 µL) containing either the test compound at the selected concentration or vehicle (DMSO; final concentration of 0.5%) was transferred to the apical chamber, and fresh, warmed media (700 µL) containing either the test compound at the selected concentration or vehicle (DMSO; final concentration of 0.5%) were transferred to the bottom chamber. After incubation for 2 hr, the media in the upper well was removed carefully. The following day, fresh, warmed media (200 µL) containing either the test compound at the selected concentration or vehicle (DMSO; final concentration of 0.5%) was transferred to the apical chamber again, and after incubation for 2 hr, the media was removed. On the third day, cells were treated again for 2 hr, and the media was removed.

The cells in the apical well were infected with 50 µL of HRV-39 (producing an MOI of approximately 10 at the estimated cell number of 1 x 10⁶/well) and incubated for 1 hr. The apical media was then removed by aspiration and the wells were washed twice with warmed PBS. The plate was incubated at 37°C. At 1 hr, and at timepoints: 10, 12, 72, and 120 hr after infection, aliquots of warmed (37°C) PBS (300 µL) were added to the apical chamber and the preparation left for 10 min. The supernatant was collected from the apical chamber. An aliquot of the supernatant (150 µL) was retained and kept at -20°C for IL-8 cytokine assay and a second aliquot (150 µL) of the supernatant was mixed with 50 µL of media containing 15% sucrose (final 3.75%) and then kept at -20°C for a virus titre assay.

IL-8 concentrations were determined using a Duoset ELISA development kit (R&D Systems, Minneapolis, MN; Figure 3B). The virus titre was estimated by CPE assay in Hela cells as follows: Supernatant (20 µL) was collected and 10-fold serial dilutions were prepared in 5%-FBS DMEM. All titrations were performed by infecting confluent Hela cell monolayers (in 96 well plates) with the serially diluted supernatant preparations (10⁻¹-10⁻⁵). The resultant cytopathic effects (CPE) were assessed by visual inspection 5 days after infection. The amount of virus required to infect 50% of Hela cells was calculated for each treatment and is reported as log[TCID₅₀] (U/20 µL).

### Measurement of kinase enzyme activities (p38α, HCK, c-SRC, SYK).

The enzyme inhibitory activity of test compounds was determined by fluorescence resonance energy transfer (FRET) using synthetic peptides labelled with both donor and acceptor fluorophores (Z-LYTE, Invitrogen). For p38 MAPK alpha (MAPK14: Invitrogen), enzyme activity was evaluated indirectly by determining the activation/phosphorylation of the downstream molecule, MAPKAP-K2. The p38 MAPK α protein was mixed with the test compound for 2hr at RT. MAPKAP-K2 (Invitrogen), the FRET peptide (2 µM), which is a phosphorylation target for MAPKAP-K2, and ATP (10 µM) were then added to the enzymes/compound mixture and incubated for 1 hr. Development reagent was added and the mixture incubated for 1 hr before detection by fluorescence completed the assay protocol. The enzymes HCK, c-SRC, and SYK were evaluated in a similar fashion. Each enzyme was incubated with the test compound for 2 hr at RT. The FRET peptides (2 µM), and appropriate ATP solutions were then added (15 µM ATP for HCK and SYK, 200 µM for c-SRC) to the enzymes/compound mixtures and incubated for 1 hr. After the development reagent was added the mixtures were incubated for 1 hr and the assay protocol was completed by detection of the fluorescence levels in a microplate reader. The percentage inhibition of each reaction was calculated relative to non-inhibited control, and the 50% inhibitory concentration (IC₅₀ value) was then calculated from the concentration-response curve.

### Measurement of HRV-16 induced activation of IRF-3 and NFκB in BEAS2B cells.

BEAS2B cells (ATCC) were infected with 10 MOI (multiplicity of infection of 10) of HRV16 and incubated for 2 hr at 33°C. The cells were then collected by scraping on ice and nuclear extracts were prepared using a nuclear protein extraction kit (ActiveMotif, Rixensart, Belgium). NFκB activity and IRF3 activity were then measured using the TransAM™ NFκB (p65) ELISA kit or the TransAM™ IRF3 ELISA kit. The effects of compounds were assessed by their addition 2 hr before HRV16 infection.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

## Claims

1. A compound or composition capable of inhibiting c-SRC and SYK activity for use in the treatment or prophylaxis of infection by human rhinovirus (HRV), wherein the compound or composition does not comprise *N*-(4-(4-(3-(3-*tert*-Butyl-1-*p*-tolyl-1H-pyrazol-5-yl) ureido)naphthalen-1-yloxy)pyridin-2-yl)-2-methoxyacetamide.

2. Use of a compound or composition capable of inhibiting c-SRC and SYK activity for the manufacture of a medicament for the treatment or prophylaxis of infection by human rhinovirus (HRV), wherein the compound or composition does not comprise *N*-(4-(4-(3-(3-*tert*-Butyl-1-*p*-tolyl-1H-pyrazol-5-yl) ureido)naphthalen-1-yloxy)pyridin-2-yl)-2-methoxyacetamide.

3. A compound, composition or use according to claim 1 or claim 2, wherein the compound or composition is administered in combination with one or more anti-viral drugs.

4. A compound, composition or use according to claim 3 wherein the one or more anti-viral drugs are selected from pleconaril and analogues thereof.

5. A method of screening for a candidate drug substance or substances intended to prevent or treat human rhinovirus (HRV) infection in a subject which comprises identifying one or more test substances which together or separately are capable of inhibiting c-SRC and SYK activity by measuring the effects of said test substance or substances on c-SRC and SYK activity.

6. A method of screening according to claim 5 comprising:
a. contacting c-SRC and SYK with a test substance in the presence of FRET peptide and ATP;
b. measuring the level of phosphorylation of FRET peptide after a set time period; and
c. comparing the level of phosphorylation measured to that observed in a control experiment in which c-SRC and SYK are not contacted with the test substance.

7. A method of screening according to claim 5 comprising:
a. contacting said substance with c-SRC and SYK or cells expressing c-SRC and SYK; and
b. determining whether c-SRC and SYK enzymatic activity is inhibited;
whereby inhibition of c-SRC and SYK enzymatic activity indicates that the substance is a candidate drug substance intended to prevent or treat human rhinovirus virus (HRV) infection

8. A compound for the use or a composition for the use or a use or method according to any one of claims 1 to 7 wherein a single compound inhibits both c-SRC and SYK activities.

9. A composition for the use or a use or method according to any one of claims 1 to 7 wherein one compound inhibits c-SRC activity and another compound inhibits SYK activity.

## Patentansprüche

1. Zum Inhibieren der c-SRC- und SYK-Aktivität fähige Verbindung oder Zusammensetzung zur Verwendung bei der Behandlung oder Prophylaxe einer Infektion durch humanen Rhinovirus (HRV), wobei die Verbindung bzw. Zusammensetzung kein *N*-(4-(4-(3-(3-*tert*.-Butyl-1-*p*-tolyl-1H-pyrazol-5-yl)ureido)naphthalin-1-yloxy)pyridin-2-yl)-2-methoxyacetamid umfasst.

2. Verwendung einer zum Inhibieren der c-SRC- und SYK-Aktivität fähigen Verbindung oder Zusammensetzung zur Herstellung eines Medikaments zur Behandlung oder Prophylaxe einer Infektion durch humanen Rhinovirus (HRV), wobei die Verbindung bzw. Zusammensetzung kein *N*-(4-(4-(3-(3-*tert*.-Butyl-1-*p*-tolyl-1H-pyrazol-5-yl)ureido)naphthalin-1-yloxy)pyridin-2-yl)-2-methoxyacetamid umfasst.

3. Verbindung, Zusammensetzung oder Verwendung nach Anspruch 1 oder Anspruch 2, wobei die Verbindung bzw. Zusammensetzung in Kombination mit einem oder mehreren antiviralen Arzneimitteln verabreicht wird.

4. Verbindung, Zusammensetzung oder Verwendung nach Anspruch 3, wobei das eine oder die mehreren antiviralen Arzneimittel aus Pleconaril und Analoga davon ausgewählt sind.

5. Verfahren zum Screenen eines Arzneimittelsubstanzkandidaten oder mehrerer Arzneimittelsubstanzkandidaten für die Prävention oder Behandlung einer Infektion durch den humanen Rhinovirus (HRV) in einem Subjekt, bei dem man eine oder mehrere zusammen oder getrennt zum Inhibieren der c-SRC- und SYK-Aktivität fähige Testsubstanzen identifiziert, indem man die Wirkungen der Testsubstanz(en) auf die c-SRC und SYK-Aktivität misst.

6. Verfahren zum Screenen nach Anspruch 5, bei dem man:
a. c-SRC und SYK in Gegenwart von FRET-Peptid und ATP mit einer Testsubstanz in Kontakt bringt,
b. den Phosphorylierungsgrad des FRET-Peptids nach einer festgelegten Zeitspanne misst und
c. den gemessenen Phosphorylierungsgrad mit dem in einem Kontrollexperiment, bei dem c-SRC und SYK nicht mit der Testsubstanz in Kontakt gebracht werden, beobachteten vergleicht.

7. Verfahren zum Screenen nach Anspruch 5, bei dem man:
a. die Substanz mit c-SRC und SYK oder c-SRC und SYK exprimierenden Zellen in Kontakt bringt und
b. feststellt, ob die enzymatische c-SRC-und SYK-Aktivität inhibiert ist,
wobei eine Inhibierung der enzymatischen c-SRC-und SYK-Aktivität darauf hindeutet, dass es sich bei der Substanz um einen Arzneimittelsubstanzkandidaten zur Prävention oder Behandlung einer Infektion durch den humanen Rhinovirus (HRV) handelt.

8. Verbindung zur Verwendung oder Zusammensetzung zur Verwendung oder Verwendung oder Verfahren nach einem der Ansprüche 1 bis 7, wobei eine einzelne Verbindung sowohl die c-SRC als auch die SYK-Aktivität inhibiert.

9. Zusammensetzung zur Verwendung oder Verwendung oder Verfahren nach einem der Ansprüche 1 bis 7, wobei eine Verbindung die c-SRC-Aktivität inhibiert und eine andere Verbindung die SYK-Aktivität inhibiert.

## Revendications

1. Composé ou composition capable d'inhiber l'activité c-SRC et SYK pour utilisation dans le traitement ou la prophylaxie d'une infection par un virus rhinovirus humain (HRV), le composé ou la composition ne comprenant pas le *N*-(4-(4-(3-(3-*tert*-butyl-1-*p*-tolyl-1H-pyrazol-5-yl)uréido)naphtalén-1-yloxy)pyridin-2-yl)-2-méthoxyacétamide.

2. Utilisation d'un composé ou d'une composition capable d'inhiber l'activité c-SRC et SYK pour la fabrication d'un médicament pour le traitement ou la prophylaxie d'une infection par un virus rhinovirus humain (HRV), le composé ou la composition ne comprenant pas le *N*-(4-(4-(3-(3-*tert*-butyl-1-*p*-tolyl-1H-pyrazol-5-yl)uréido)naphtalén-1-yloxy)pyridin-2-yl)-2-méthoxyacétamide.

3. Composé, composition ou utilisation selon la revendication 1 ou la revendication 2, le composé ou la composition étant administré en combinaison avec un ou plusieurs médicaments antiviraux.

4. Composé, composition ou utilisation selon la revendication 3, les un ou plusieurs médicaments antiviraux étant choisis parmi le pléconaril et des analogues de celui-ci.

5. Procédé de criblage d'une substance ou de substances pharmaceutique(s) candidate(s) destinée(s) à prévenir ou traiter une infection par un virus rhinovirus humain (HRV) chez un sujet qui comprend l'identification d'une ou plusieurs substances d'essai qui, conjointement ou séparément, sont capables d'inhiber l'activité c-SRC et SYK par mesure des effets de ladite substance ou desdites substances d'essai sur l'activité c-SRC et SYK.

6. Procédé de criblage selon la revendication 5 comprenant :
a. la mise en contact de c-SRC et SYK avec une substance d'essai en présence de peptide FRET et d'ATP ;
b. la mesure du taux de phosphorylation de peptide FRET après une durée définie ; et
c. la comparaison du taux de phosphorylation mesuré à celui observé dans un essai témoin dans lequel c-SRC et SYK ne sont pas mis en contact avec la substance d'essai.

7. Procédé de criblage selon la revendication 5 comprenant :
a. la mise en contact de ladite substance avec c-SRC et SYK ou des cellules exprimant c-SRC et SYK ; et
b. la détermination du fait que l'activité enzymatique c-SRC et SYK est inhibée ou non ;
dans lequel l'inhibition de l'activité enzymatique c-SRC et SYK indique que la substance est une substance pharmaceutique candidate destinée à prévenir ou traiter une infection par un virus rhinovirus humain (HRV).

8. Composé pour l'utilisation ou composition pour l'utilisation ou utilisation ou procédé selon l'une quelconque des revendications 1 à 7, un composé unique inhibant à la fois les activités c-SRC et SYK.

9. Composition pour l'utilisation ou utilisation ou procédé selon l'une quelconque des revendications 1 à 7, un composé inhibant l'activité c-SRC et un autre composé inhibant l'activité SYK.
